(19) Europäisches Patentamt
European Patent Office
Office européen des brevets

(11) EP 4 240 315 B1

(12) # EUROPÄISCHE PATENTSCHRIFT

(45) Veröffentlichungstag und Bekanntmachung des Hinweises auf die Patenterteilung:
**27.08.2025 Patentblatt 2025/35**

(21) Anmeldenummer: **21791307.8**

(22) Anmeldetag: **11.10.2021**

(51) Internationale Patentklassifikation (IPC):
***A61K 8/39*** *(2006.01)* ***A61K 8/898*** *(2006.01)*
***A61Q 5/06*** *(2006.01)*

(52) Gemeinsame Patentklassifikation (CPC):
**A61K 8/39; A61K 8/898; A61Q 5/065;**
A61K 2800/43

(86) Internationale Anmeldenummer:
**PCT/EP2021/078041**

(87) Internationale Veröffentlichungsnummer:
**WO 2022/096232 (12.05.2022 Gazette 2022/19)**

(54) **MITTEL ZUM FÄRBEN VON KERATINISCHEM MATERIAL MIT AMINOSILIKON, FARBGEBENDER VERBINDUNG UND EINEM ANLAGERUNGSPRODUKT VON C1-C6 ALKYLENOXID(EN) AN DIE ESTER AUS FETTSÄUREN UND AROMATISCHEN ALKOHOLEN**

AGENT FOR DYEING KERATINOUS MATERIAL, COMPRISING AMINOSILICONE, A CHROMOPHORIC COMPOUND AND AN ADDITION PRODUCT OF C1-C6 ALKYLENE OXIDE(S) TO THE ESTERS OF FATTY ACIDS AND AROMATIC ALCOHOLS

AGENT DE COLORATION DE MATIÈRES KÉRATINIQUES, COMPRENANT DE L'AMINOSILICONE, UN COMPOSÉ CHROMOPHORE ET UN PRODUIT D'ADDITION D'OXYDE(S) D'ALKYLÈNE C1-C6 AUX ESTERS D'ACIDES GRAS ET D'ALCOOLS AROMATIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.11.2020 DE 102020213785**

(43) Veröffentlichungstag der Anmeldung:
**13.09.2023 Patentblatt 2023/37**

(73) Patentinhaber: **Henkel AG & Co. KGaA**
**40589 Düsseldorf (DE)**

(72) Erfinder:
- **KRUCK, Constanze**
  **41515 Grevenbroich (DE)**
- **HILBIG, Sandra**
  **44894 Bochum (DE)**
- **MOCH, Melanie**
  **41542 Dormagen (DE)**
- **KESSLER-BECKER, Daniela**
  **51371 Leverkusen (DE)**

(56) Entgegenhaltungen:
**EP-A2- 2 441 434** **DE-A1- 102008 037 633**
**US-A1- 2013 149 358**

- **DATABASE GNPD [online] MINTEL; 13 August 2020 (2020-08-13), ANONYMOUS: "Gloss Semi-Permanent Treatment Gloss", XP055878859, retrieved from https://www.gnpd.com/sinatra/recordpage/7997249/ Database accession no. 7997249**
- **DATABASE GNPD [online] MINTEL; 19 July 2019 (2019-07-19), ANONYMOUS: "Shade Shot Gloss", XP055878952, retrieved from https://www.gnpd.com/sinatra/recordpage/6691599/ Database accession no. 6691599**
- **DATABASE GNPD [online] MINTEL; 4 August 2011 (2011-08-04), ANONYMOUS: "Three-Dimensional Shine Hair Tint", XP055878965, retrieved from https://www.gnpd.com/sinatra/recordpage/1602139/ Database accession no. 1602139**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Processed by Luminess, 75001 PARIS (FR)

## Beschreibung

Gegenstand der vorliegenden Anmeldung ist ein Mittel zum Färben von keratinischem Material, insbesondere menschlichen Haaren, welches mindestens ein aminofunktionalisiertes Silikonpolymer (a1), mindestens eine farbgebende Verbindung (a2) sowie mindestens ein Anlagerungsprodukt von $C_1$-$C_6$-Alkylenoxid(en) an die Ester aus Fettsäuren und aromatischen Alkoholen enthält.

Ein zweiter Gegenstand dieser Anmeldung ist ein Verfahren zum Färben von keratinischem Material, insbesondere menschlichen Haaren, wobei ein Mittel des ersten Erfindungsgegenstands auf dem keratinischen Material angewendet, einwirken gelassen und danach wieder mit Wasser ausgewaschen wird.

Die Veränderung von Form und Farbe von keratinischem Material, insbesondere von menschlichen Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit tensidhaltigen Reinigungsmitteln wieder rückstandslos entfernen. Unter dem Namen Haar-Mascara sind verschiedene Produkte dieses Typs auf dem Markt erhältlich.

Wünscht sich der Anwender besonders langanhaltende Färbungen, so ist die Verwendung von oxidativen Färbemitteln bislang seine einzige Option. Doch trotz vielfacher Optimierungsversuche lässt sich bei der oxidativen Haarfärbung ein unangenehmer Ammoniakgeruch bzw. Amingeruch nicht vollständig vermeiden. Auch die mit dem Einsatz der oxidativen Färbemittel nach wie vor verbundene Haarschädigung wirkt sich auf das Haar des Anwenders nachteilig aus. Eine nach wie vor bestehende Herausforderung ist daher die Suche nach alternativen, leistungsstarken Färbeverfahren. Aus diesem Grund liegen auf Pigmenten basierende Färbemittel stark im Trend. Jedoch sind vor allem die Farbintensitäten und Echtheitseigenschaften von Färbemitteln, die auf dem Einsatz von Pigmenten basieren, noch stark verbesserungswürdig.

Mintel GNPD Record ID 7997249, "Gloss Semi-Permanent Treatment Gloss", August 2020 und Mintel GNPD Record ID 6691599, "Shade Shot Gloss", Juli 2019 zeigen beide eine Mittel zum Färben von keratinischem Material, insbesondere menschlichen Haaren, enthaltend (a1) mindestens ein aminofunktionalisiertes Silikonpolymer (Bis-Cetearyl Amodimethicone), (a2) mindestens eine farbgebende Verbindung (Basic Red 51, Basic Brown 17, HC Blue No.15), und (a3) mindestens ein Anlagerungsprodukt von C1-C6 -Alkylenoxid(en) an die Ester aus C12 -C30-Fettsäuren und aromatischen C1-C12 -Alkoholen (PPG-3 Benzyl Ether Myristate). Das Mittel wird auf das Haar angewendet, zwischen 10 und 20 Minuten einwirken lassen und danach ausgespült.

Es war die Aufgabe der vorliegenden Erfindung, ein Färbmittel bereitzustellen, das es ermöglicht, Pigmente in extrem dauerhafter Weise auf den Haaren zu fixieren. Bei Anwendung des Mittels in einem Färbeverfahren sollten besonders intensive Färbeergebnisse erzielt werden. Weiterhin sollten auch Färbungen mit einer guten Waschechtheit, einem guten Egalisiervermögen und einem besonders gleichmäßigen Farbresultat erzielt werden. Zudem sollte auch der Abrieb dieser Pigment-Färbemittel besonders gering sein, d.h. auch bei mechanischem Kontakt mit der Kleidung oder anderen Textilien sollten die Färbemittel eine besonders gute Reibechtheit besitzen. Darüber hinaus sollten das Keratinmaterial, insbesondere die Haare, sich nach Anwendung der Pigment-Färbemittel weder beschwert noch stumpf oder fettig anfühlen und einen möglichst angenehmen Griff aufweisen. Überraschenderweise hat sich nun herausgestellt, dass die vorgenannte Aufgabe hervorragend gelöst werden kann, wenn keratinische Materialien, insbesondere Haare, mit einem Mittel gefärbt werden, welches mindestens ein aminofunktionalisiertes Silikonpolymer (a1), mindestens eine farbgebende Verbindung (a2), und mindestens ein Anlagerungsprodukt von $C_1$-$C_6$-Alkylenoxid(en) an die Ester aus $C_{12}$-$C_{30}$-Fettsäuren und aromatischen $C_1$-$C_{12}$-Alkoholen (a3) enthält.

Ein erster Gegenstand der vorliegenden Erfindung ist ein Mittel zum Färben von keratinischem Material, insbesondere menschlichen Haaren, enthaltend

(a1) mindestens ein aminofunktionalisiertes Silikonpolymer, und
(a2) mindestens eine farbgebende Verbindung,

(a3) mindestens ein Anlagerungsprodukt von $C_1$-$C_6$-Alkylenoxid(en) an die Ester aus $C_{12}$-$C_{30}$-Fettsäuren und aromatischen $C_1$-$C1_2$-Alkoholen, und
(a4) mindestens ein Anlagerungsprodukt von $C_1$ -$C_6$ -Alkylenoxid(en) an aliphatische $C_1$-$C_{24}$ -Alkanole der Formel (AA-I).

Im Zuge der zu dieser Erfindung führenden Arbeiten hat sich überraschenderweise gezeigt, dass der Einsatz von alkoxylierte Verbindungen (a3) und (a4) in einem Mittel, welches ein Aminosilikon (a1) sowie eine farbgebende Verbindung (a2) enthält, zu einer Verbesserung der Farbintensität und der Waschechtheit führt, wenn dieses Mittel in einem Färbeverfahren auf dem keratinischen Material, insbesondere auf menschlichen Haaren, angewendet wird. Diese positiven Effekte wurden insbesondere dann beobachtet, wenn es sich der farbgebenden Verbindungen (a2) um ein Pigment handelt.

<u>keratinisches Material</u>

Unter keratinischem Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.
Bevorzugt werden unter keratinischem Material das menschliche Haar, die menschliche Haut und menschliche Nägel, insbesondere Finger- und Fußnägel, verstanden. Ganz besonders bevorzugt wird unter keratinischem Material das menschliche Haar verstanden.

<u>Mittel zur Färbung</u>

Der Begriff "Mittel zur Färbung" wird im Rahmen dieser Erfindung für eine durch Einsatz von farbgebenden Verbindungen, insbesondere Pigmenten, hervorgerufene Farbgebung des Keratinmaterials, insbesondere des Haares, verwendet. Bei dieser Färbung lagern sich die Pigmente als farbgebende Verbindungen in einem besonders homogenen, gleichmäßigen und glatten Film an der Oberfläche des Keratinmaterials ab.
Das erfindungsgemäße Mittel enthält die erfindungswesentlichen Bestandteile (a1), (a2), (a3) und (a4), bevorzugt in einem kosmetischen Träger.

<u>aminofunktionalisierte Silikonpolymere (a1)</u>

Als ersten erfindungswesentlichen Inhaltsstoff (a1) enthält das Mittel mindestens ein aminofunktionalisiertes Silikonpolymer. Das aminofunktionalisiertes Silikonpolymer kann alternativ auch als Aminosilikon oder Amodimethicone bezeichnet werden.
Silikonpolymere sind im allgemeinen Makromoleküle mit einem Molekulargewicht von mindestens 500 g/mol, bevorzugt mindestens 1000 g/mol, weiter bevorzugt von mindestens 2500 g/mol, besonders bevorzugt von mindestens 5000 g/mol, welche sich wiederholende organische Einheiten umfassen.
Das maximale Molekulargewicht des Silikonpolymers hängt von dem Polymerisationsgrad (Anzahl der polymerisierten Monomere) und der Ansatzgröße ab und wird durch die Polymerisationsmethode mitbestimmt. Im Sinne der vorliegenden Erfindung ist es bevorzugt, wenn das maximale Molekulargewicht des Silikonpolymers nicht mehr als $10^7$ g/mol, bevorzugt nicht mehr als $10^6$ g/mol und besonders bevorzugt nicht mehr als $10^5$ g/mol beträgt.
Die Silikonpolymere umfassen viele Si-O-Wiederholungseinheiten, wobei die Si-Atome organische Reste wie beispielsweise Alkylgruppen oder substituierte Alkylgruppen tragen können. Alternativ wird ein Silikonpolymer daher auch als Polydimethylsiloxan bezeichnet.
In Entsprechung des hohen Molekulargewichts der Silikonpolymere basieren diese auf mehr als 10 Si-O Wiederholungseinheiten, bevorzugt mehr als 50 Si-O-Wiederholungseinheiten und besonders bevorzugt mehr als 100 Si-O-Wiederholungseinheiten, ganz besonders bevorzugt mehr als 500 Si-O-Wiederholungseinheiten.
Unter einem aminofunktionalisierten Silikonpolymer wird ein funktionalisiertes Silikon verstanden, welches mindestens eine Struktureinheit mit einer Aminogruppe trägt. Bevorzugt trägt das aminofunktionalisierte Silikonpolymer mehrere Struktureinheiten mit jeweils mindestens einer Aminogruppe. Unter einer Aminogruppe wird eine primäre Aminogruppe, eine sekundäre Aminogruppe und eine tertiäre Aminogruppe verstanden. Alle diese Aminogruppen können im sauren Milieu protoniert werden und liegen dann in ihrer kationischen Form vor.
Prinzipiell konnten gute Effekte mit aminofunktionalisierten Silikonpolymeren (a1) erzielt werden, wenn diese mindestens eine primäre, mindestens eine sekundäre und/oder mindestens eine tertiäre Aminogruppe tragen. Färbungen mit der besten Waschechtheit wurden jedoch beobachtet, wenn ein aminofunktionalisiertes Silikonpolymer (a1) im Mittel eingesetzt wurde, welches mindestens eine sekundäre Aminogruppe enthält.
In einer ganz besonders bevorzugten Ausführungsform ist ein erfindungsgemäßes Mittel dadurch gekennzeichnet, dass es mindestens ein aminofunktionalisiertes Silikonpolymer (a1) mit mindestens eine sekundären Aminogruppe enthält.

Die sekundäre Aminogruppe(n) kann bzw. können sich an verschiedenen Positionen des aminofunktionalisierten Silikonpolymers befinden. Ganz besonders gute Effekt wurden gefunden, wenn ein aminofunktionalisiertes Silikonpolymer (a1) eingesetzt wurde, dass mindestens eine, bevorzugt mehrere Struktureinheiten der Formel (Si-Amino) besitzt.

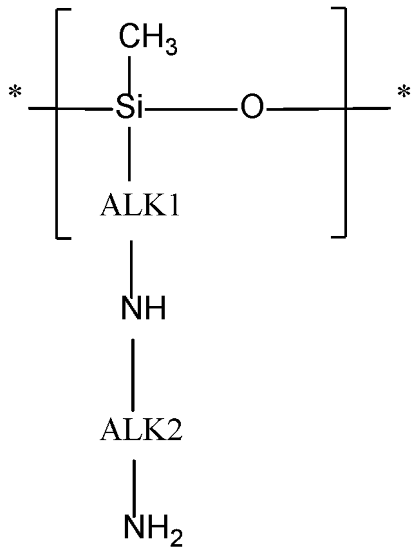

(Si-Amino)

In den Struktureinheiten der Formel (Si-Amino) steht die Kürzel ALK1 und ALK2 unabhängig voneinander für eine lineare oder verzweigte, zweiwertige $C_1$-$C_{20}$-Alkylengruppe.

In einer weiteren ganz besonders bevorzugten Ausführungsform ist ein erfindungsgemäßes Mittel dadurch gekennzeichnet, dass es mindestens ein aminofunktionalisiertes Silikonpolymer (a1) enthält, das mindestens eine Struktureinheit der Formel (Si-Amino) umfasst,

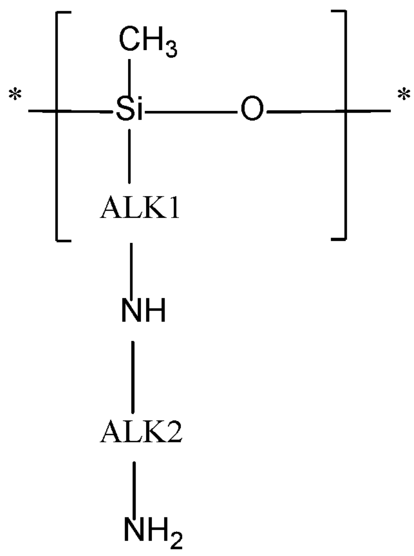

(Si-Amino)

wobei

ALK1 und ALK2 unabhängig voneinander für eine lineare oder verzweigte, zweiwertige $C_1$-$C_{20}$-Alkylengruppe stehen.

Die mit einem Stern (*) gekennzeichneten Position geben hierbei jeweils die Bindung zu weiteren Struktureinheiten des Silikonpolymers an. Beispielsweise kann das dem Stern benachbarte Silicium-Atom an ein weiteres Sauerstoffatom gebunden sein, und das dem Stern benachbarte Sauerstoffatom kann an ein weiteres Siliciumatom oder auch

an eine $C_1$-$C_6$-Alkylgruppe gebunden sein.

Eine zweiwertige $C_1$-$C_{20}$-Alkylengruppe kann alternativ auch als eine divalente oder zweibindige $C_1$-$C_{20}$-Alkylengruppe bezeichnet werden, womit gemeint ist, dass jede Gruppierung ALK1 bzw. AK2 zwei Bindungen eingehen kann.

Im Fall von ALK1 erfolgt eine Bindung vom Silicium-Atom zur Gruppierung ALK1, und die zweite Bindung besteht zwischen ALK1 und der sekundären Aminogruppe.

Im Fall von ALK2 erfolgt eine Bindung von der sekundären Aminogruppe zur Gruppierung ALK2, und die zweite Bindung besteht zwischen ALK2 und der primären Aminogruppe.

Beispiele für eine lineare zweiwertige $C_1$-$C_{20}$-Alkylengruppe sind beispielsweise die Methylen-gruppe ($-CH_2-$), die Ethylengruppe ($-CH_2-CH_2-$), die Propylengruppe ($-CH_2-CH_2-CH_2-$) und die Butylengruppe ($-CH_2-CH_2-CH_2-CH_2-$). Die Propylengruppe ($-CH_2-CH_2-CH_2-$) ist besonders bevorzugt. Ab einer Kettenlänge von 3 C-Atomen können zweiwertige Alkylengruppen auch verzweigt sein. Beispiele für verzweigte, zweiwertige $C_3$-$C_{20}$-Alkylengruppen sind ($-CH_2-CH(CH_3)-$) und ($-CH_2-CH(CH_3)-CH_2-$).

In einer weiteren besonders bevorzugten Ausführungsform stellen die Struktureinheiten der Formel (Si-Amino) Wiederholungseinheiten im aminofunktionalisierten Silikonpolymer (a1) dar, so dass das Silikonpolymer mehrer Struktureinheiten der Formel (Si-Amino) umfasst.

Im Folgenden werden besonders gut geeignete aminofunktionalisierte Silikonpolymere (a1) mit mindestens einer sekundären Aminogruppe aufgelistet.

Färbungen mit den allerbesten Waschechtheiten konnten erhalten werden, wenn im erfindungsgemäßen Verfahren mindestens ein Mittel auf dem keratinischen Material appliziert wurde, das mindestens ein aminofunktionalisiertes Silikonpolymer (a1) enthält, das Struktureinheiten der Formel (Si-I) und der Formel (Si-II) umfasst

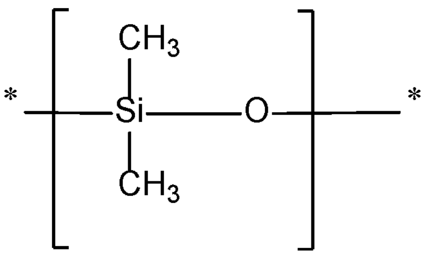

(Si-I)

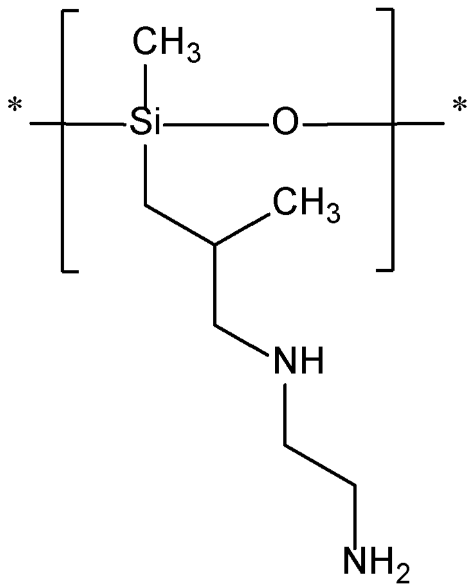

(Si-II).

In einer weiteren explizit ganz besonders bevorzugten Ausführungsform ist ein erfindungsgemäßes Mittel dadurch gekennzeichnet, dass es mindestens ein aminofunktionalisiertes Silikonpolymer (a1) enthält, das Struktureinheiten der Formel (Si-I) und der Formel (Si-II) umfasst

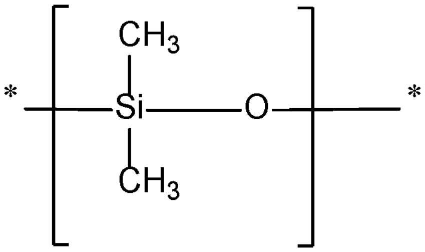

(Si-I)

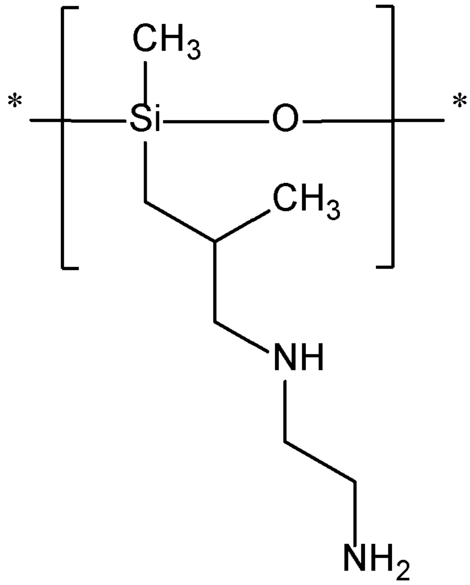

(Si-II).

Ein entsprechendes aminofunkionalisiertes Silikonpolymer mit den Struktureinheiten (Si-I) und (Si-II) ist beispielweise das Handelsprodukt DC 2-8566 bzw. Dowsil 2-8566 Amino Fluid, das von der Firma Dow Chemical Company komerziell vertrieben wird und welches die Benennung "Siloxanes and Silicones, 3-[(2-Aminoethyl)amino]-2-methylpropyl Me, Di-Me-Siloxane" sowie die CAS-Nummer 106842-44-8 trägt. Ein weiteres aminofunkionalisiertes Silikonpolymer mit den Struktureinheiten (Si-I) und (Si-II) ist beispielweise das Handelsprodukt DOWSIL™ AP-8568 Amino Fluid, das ebenfalls das von der Firma Dow Chemical Company komerziell vertrieben wird.

Im Rahmen einer weiteren bevorzugten Ausführungsform ist ein erfindungsgemäßes Mittel dadurch gekennzeichnet, dass es mindestens ein aminofunktionelles Silikonpolymer (a1) der Formel der Formel (Si-III) enthält,

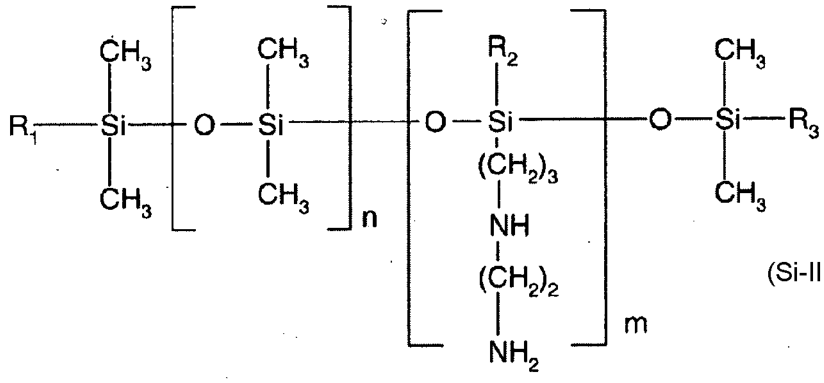

(Si-III)

wobei

- m und n bedeuten Zahlen, die so gewählt sind, daß die Summe (n + m) im Bereich von 1 bis 1000 liegt,
- n ist eine Zahl im Bereich von 0 bis 999 und m ist eine Zahl im Bereich von 1 bis 1000,
- R1, R2 und R3, die gleich oder verschieden sind, bedeuten eine Hydroxygruppe oder eine C1-4-Alkoxygruppe,
- wobei mindestens eine der Gruppen R1 bis R3 eine Hydroxygruppe bedeutet;

Ein weiteres erfindungsgemäß bevorzugtes Mittel ist dadurch gekennzeichnet, dass es mindestens aminofunktionelles Silikonpolymer (a1) der Formel der Formel (Si-IV) enthält,

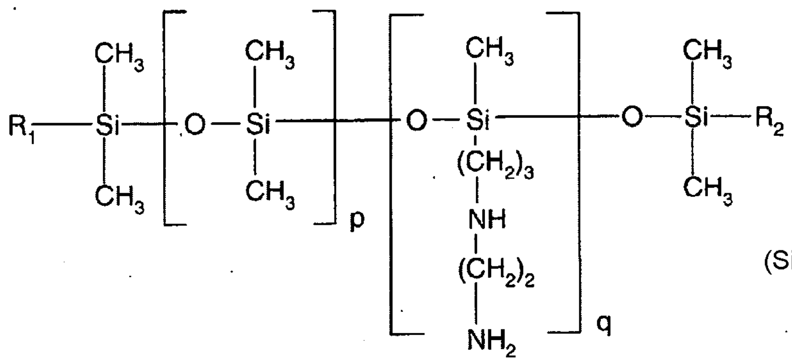

(Si-IV)

in der

- p und q bedeuten Zahlen, die so gewählt sind, daß die Summe (p + q) im Bereich von 1 bis 1000 liegt,
- p ist eine Zahl im Bereich von 0 bis 999 und q ist eine Zahl im Bereich von 1 bis 1000,
- R1 und R2, die verschieden sind, bedeuten eine Hydroxygruppe oder eine C1-4-Alkoxygruppe, wobei mindestens eine der Gruppen R1 bis R2 eine Hydroxygruppe bedeutet.

Die Silikone der Formeln (Si-III) und (Si-IV) unterscheiden sich durch die Gruppierung am Si-Atom, das die stickstoffhaltige Gruppe trägt: In Formel (Si-III) bedeutet R2 eine Hydroxygruppe oder eine C1-4-Alkoxygruppe, während der Rest in Formel (Si-IV) eine Methylgruppe ist. Die einzelnen Si-Gruppierungen, die mit den Indices m und n bzw. p und q gekennzeichnet sind, müssen nicht als Blöcke vorliegen, vielmehr können die einzelnen Einheiten auch statistisch verteilt vorliegen, d.h. in den Formeln (Si-III) und (Si-IV) ist nicht zwingend jedes R1-Si$(CH_3)_2$-Gruppe an eine -[O-Si$(CH_3)_2$]-Gruppierung gebunden.

Als besonders wirkungsvoll im Hinblick auf die gewünschten Effekte haben sich auch erfindungsgemäßen Mittel erwiesen, welche mindestens ein aminofunktionelles Silikonpolymer (a1) der Formel der Formel (Si-V) enthalten

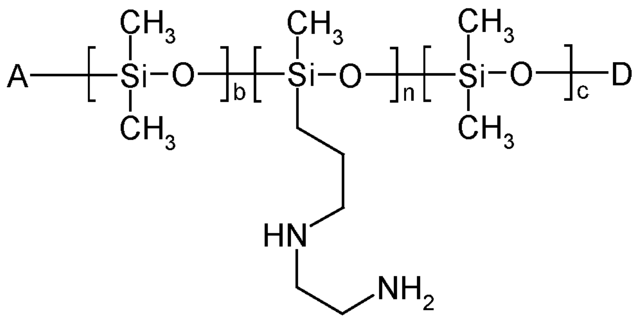

(Si-V),

in der

| | |
|---|---|
| A | für eine Gruppe -OH, -O-Si$(CH_3)_3$,-O-Si$(CH_3)_2$OH ,-O-Si$(CH_3)_2$$OCH_3$ steht, |
| D | für eine Gruppe -H, -Si$(CH_3)_3$,-Si$(CH_3)_2$OH, -Si$(CH_3)_2$$OCH_3$ steht, |
| b, n und c | für ganze Zahlen zwischen 0 und 1000 stehen, mit den Maßgaben |

- $n > 0$ und $b + c > 0$
- mindestens eine der Bedingungen A = -OH bzw. D = -H ist erfüllt.

In der vorstehend genannten Formel (Si-V) sind die einzelnen Siloxaneinheiten mit den Indices b, c und n statistisch verteilt, d.h. es muß sich nicht zwingend um Blockcopolymere handeln.

Das Mittel kann weiterhin auch ein oder mehrere verschiedene aminofunktionalisierte Silikonpolymere enthalten, die durch die Formel (Si-VI)

$$M(R_aQ_bSiO_{(4-a-b)/2)x}(R_cSiO_{(4-c)/2)y}M \qquad \text{(Si-VI)}$$

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel $-R^1HZ$ ist, worin $R^1$ eine zweiwertige, verbindende

Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stand der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von $R^1$ schließen Methylen, Ethylen, Propylen, Hexamethylen, Decamethylen, -$CH_2CH(CH_3)CH_2$-, Phenylen, Naphthylen, -$CH_2CH_2SCH_2CH_2$-, -$CH_2CH_2OCH_2$-, -$OCH_2CH_2$-, -$OCH_2CH_2CH_2$-, -$CH_2CH(CH_3)C(O)OCH_2$-, -$(CH_2)_3CC(O)OCH_2CH_2$-, -$C_6H_4C_6H_4$-, -$C_6H_4CH_2C_6H_4$-; und -$(CH_2)_3C(O)SCH_2CH_2$- ein.

Z ist ein organischer, aminofunktioneller Rest, enthaltend mindestens eine funktionelle Aminogruppe. Eine mögliche Formel für Z ist $NH(CH_2)_zNH_2$, worin z 1 oder mehr ist. Eine andere mögliche Formel für Z ist -$NH(CH_2)_z(CH_2)_{zz}NH$, worin sowohl z als auch zz unabhängig 1 oder mehr sind, wobei diese Struktur Diamino-Ringstrukturen umfaßt, wie Piperazinyl. Z ist am bevorzugtesten ein -$NHCH_2CH_2NH_2$-Rest. Eine andere mögliche Formel für Z ist - $N(CH_2)_z(CH_2)_{zz}NX_2$ oder -$NX_2$, worin jedes X von $X_2$ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel -$CH_2CH_2CH_2NHCH_2CH_2NH_2$. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der $R_aQ_bSiO_{(4-a-b)/2}$-Einheiten zu den $R_cSiO_{(4-c)/2}$-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Im Rahmen einer besonders bevorzugten Ausführungsform ist ein erfindungsgemäßes Mittel dadurch gekennzeichnet, dass es midnestens ein aminofunktionelles Silikonpolymer der Formel (Si-VII)

$$R'_aG_{3-a}\text{-}Si(OSiG_2)_n\text{-}(OSiG_bR'_{2-b})_m\text{-}O\text{-}SiG_{3-a}\text{-}R'_a \qquad \text{(Si-VII)},$$

enthält, worin bedeutet:

- G ist-H, eine Phenylgruppe, -OH, -O-$CH_3$, -$CH_3$, -O-$CH_2CH_3$, -$CH_2CH_3$, -O-$CH_2CH_2CH_3$,-$CH_2CH_2CH_3$, -O-$CH(CH_3)_2$, -$CH(CH_3)_2$, -O-$CH_2CH_2CH_2CH_3$, - $CH_2CH_2CH_2CH_3$, -O-$CH_2CH(CH_3)_2$, -$CH_2CH(CH_3)_2$, -O-$CH(CH_3)CH_2CH_3$, - $CH(CH_3)CH_2CH_3$, -O-$C(CH_3)_3$, -$C(CH_3)_3$ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus

  ◦ -Q-N(R")-$CH_2$-$CH_2$-N(R")$_2$
  ◦ -Q-N(R")$_2$
  ◦ -Q-N'(R")$_3A^-$
  ◦ -Q-$N^+$H(R")$_2$ $A^-$
  ◦ -Q-N'$H_2$(R")$A^-$
  ◦ -Q-N(R")-$CH_2$-$CH_2$-N'R"$H_2A^-$,

  wobei jedes Q für eine chemische Bindung, -$CH_2$-, -$CH_2$-$CH_2$-, -$CH_2CH_2CH_2$-, -$C(CH_3)_2$-, -$CH_2CH_2CH_2CH_2$-, -$CH_2C(CH_3)_2$-, -$CH(CH_3)CH_2CH_2$- steht,
  R" für gleiche oder verschiedene Reste aus der Gruppe -H, -Phenyl, -Benzyl, -$CH_2$-$CH(CH_3)$Ph, der $C_{1-20}$-Alkylreste, vorzugsweise -$CH_3$, -$CH_2CH_3$, -$CH_2CH_2CH_3$, - $CH(CH_3)_2$, -$CH_2CH_2CH_2H_3$, -$CH_2CH(CH_3)_2$, -$CH(CH_3)$

$CH_2CH_3$, $-C(CH_3)_3$, steht und A ein Anion repräsentiert, welches vorzugsweise ausgewählt ist aus Chlorid, Bromid, Iodid oder Methosulfat.

Im Rahmen einer weiteren bevorzugten Ausführungsform ist ein erfindungsgemäßes Mittel dadurch, dass es mindestens ein aminofunktionelles Silikonpolymer (a1) der Formel (Si-VIIa) enthält,

$$(CH_3)_3Si\text{-}[O\text{-}Si(CH_3)_2]_n[OSi(CH_3)]_m\text{-}OSi(CH_3)_3 \qquad \text{(Si-VIIa),}$$

$$|$$

$$CH_2CH(CH_3)CH_2NH(CH_2)_2NH_2$$

worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet.

Im Rahmen einer weiteren bevorzugten Ausführungsform ist ein erfindungsgemäßes Mittel dadurch gekennzeichnet, dass es mindestens ein aminofunktionelles Silikonpolymer (a1) der Formel (Si-VIIb) enthält

$$R\text{-}[Si(CH_3)_2\text{-}O]_{n1}[Si(R)\text{-}O]_m\text{-}[Si(CH_3)_2]_{n2}\text{-}R \qquad \text{(Si-VIIb),}$$

$$|$$

$$(CH_2)_3NH(CH_2)_2NH_2$$

enthalten, worin R für -OH, $-O-CH_3$ oder eine $-CH_3$-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese aminofunktionalisierten Siliconpolymere werden nach der INCI-Deklaration als Amodimethicone bezeichnet.

Unabhängig davon, welche aminofunktionellen Silicone eingesetzt werden, sind erfindungsgemäße Mittel bevorzugt, die ein aminofunktionelles Silikonpolymer enthalten, dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden.

Weiterhin sind auch Mittel geeignet, welche ein spezielles 4-Morpholinomethyl-substituiertes Silikonpolymer (a1) enthielten. Dieses aminofunktionalisierte Silikonpolymer umfasst Struktureinheiten der Formeln (SI-VIII) und der Formel (Si-IX)

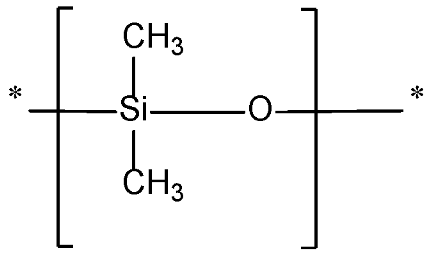

(Si-VIII)

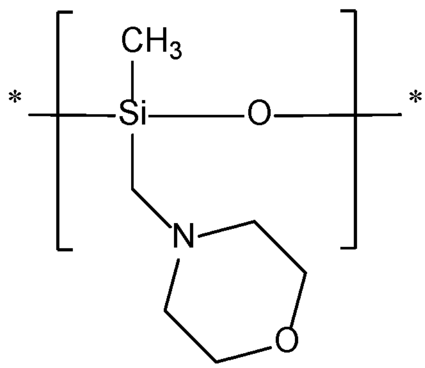

(Si-IX).

.Entsprechende 4-Morpholinomethyl-substituiertes Silikonpolymere werden im folgenden beschrieben.

Ein ganz besonders bevorzugtes aminofunktionaliserte Silikonpolymer ist unter dem Namen Amodimethicone/Morpholinomethyl Silsesquioxane Copolymer bekannt und in Form des Rohstoffes Belsil ADM 8301 E von Wacker kommerziell erhältlich.

Als 4-morpholinomethyl-substituiertes Silikon kann beispielsweise ein Silikon eingesetzt werden, welches

Struktureinheiten der Formeln (Si-VIII), (Si-IX) und (Si-X) aufweist

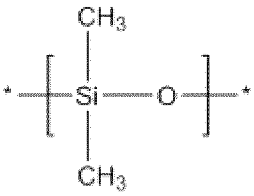
(Si-VIII),
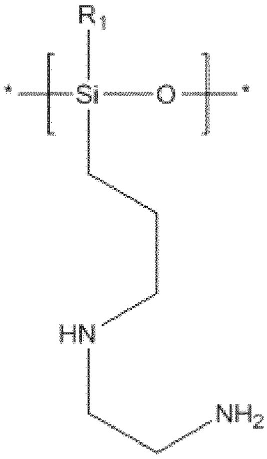
(Si-X)
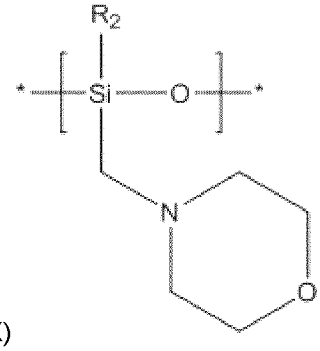
(Si-IX)

in denen

R1 für $-CH_3$, -OH, $-OCH_3$, $-O-CH_2CH_3$, $-O-CH_2CH_2CH_3$, oder $-O-CH(CH_3)_2$ steht;
R2 für $-CH_3$, -OH, oder $-OCH_3$ steht.

Besonders bevorzugte erfindungsgemäße Mittel enthalten mindestens ein 4-morpholinomethylsubstituierten Silikons der Formel (Si-XI)

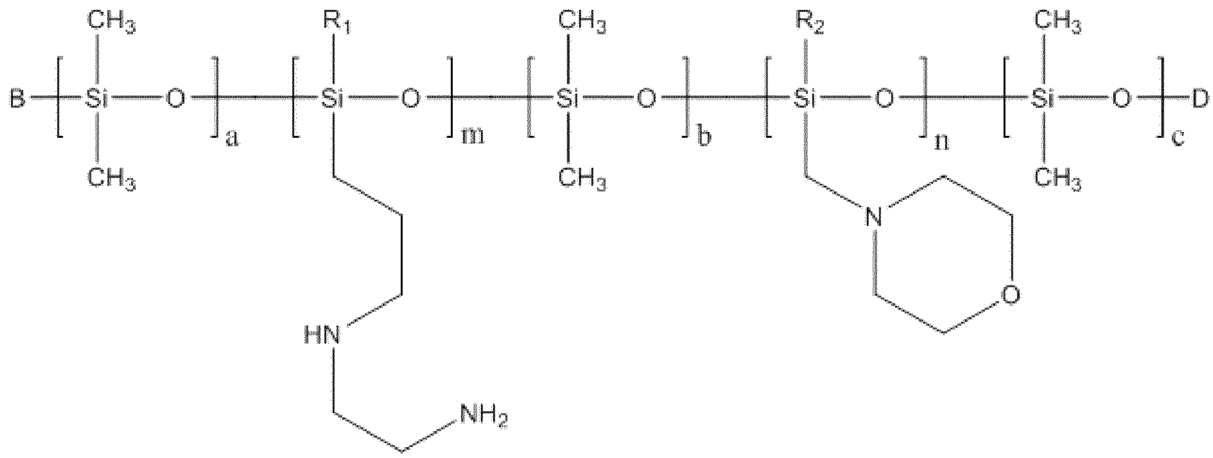

(Si-XI)

in der

R1 für $-CH_3$, -OH, $-OCH_3$, $-O-CH_2CH_3$, $-O-CH_2CH_2CH_3$, oder $-O-CH(CH_3)_2$ steht;
R2 für $-CH_3$, -OH, oder $-OCH_3$ steht.
B für eine Gruppe -OH, $-O-Si(CH_3)_3$,$-O-Si(CH_3)_2OH$ ,$-O-Si(CH_3)_2OCH_3$ steht,
D für eine Gruppe -H, $-Si(CH_3)_3$,$-Si(CH_3)_2OH$, $-Si(CH_3)_2OCH_3$ steht,
a, b und c unabhängig voneinander für ganze Zahlen zwischen 0 und 1000 stehen, mit der Maßgabe $a + b + c > 0$
m und n unabhängig voneinander für ganze, Zahlen zwischen 1 und 1000 stehen mit den Maßgabe, daß

- mindestens eine der Bedingungen B = -OH bzw. D = -H erfüllt ist,
- die Einheiten a, b, c, m und n statistisch oder blockweise im Molekül verteilt vorliegen.

Strukturformel (Si-XI) soll verdeutlichen, daß die Siloxangruppen n und m nicht zwingend direkt an eine Endgruppierung B bzw. D gebunden sein müssen. Vielmehr gilt in bevorzugten Formeln (Si-VI) $a > 0$ oder $b > 0$ und

in besonders bevorzugten Formeln (Si-VI) a > 0 und c > 0, d.h. die terminale Gruppierung B bzw. D ist vorzugsweise an eine Dimethylsiloxy-Gruppierung gebunden. Auch in Formel (Si-VI) sind die Siloxaneinheiten a, b, c, m und n vorzugsweise statistisch verteilt.

Die durch Formel (Si-VI) dargestellten erfindungsgemäß eingesetzten Silikone können trimethylsilylterminiert sein (D oder B = $-Si(CH_3)_3$), sie können aber auch zweiseitig dimethylsilylhydroxy- oder einseitig dimethylsilylhydroxy- und dimethylsilylmethoxy-terminiert sein. Im Rahmen der vorliegenden Erfindung besonders bevorzugt eingesetzte Silikone sind ausgewählt aus Silikonen, in denen

B = $-O-Si(CH_3)_2OH$ und D = $-Si(CH_3)_3$

B = $-O-Si(CH_3)_2OH$ und D = $-Si(CH_3)_2OH$

B = $-O-Si(CH_3)_2OH$ und D = $-Si(CH_3)_2OCH_3$

B = $-O-Si(CH_3)_3$ und D = $-Si(CH_3)_2OH$

B = $-O-Si(CH_3)_2OCH_3$ und D = $-Si(CH_3)_2OH$

bedeutet. Diese Silikone führen zu exorbitanten Verbesserungen der Haareigenschaften der mit den erfindungsgemäßen Mitteln behandelten Haare, und zu einem gravierend verbesserten Schutz bei oxidativer Behandlung.

Es hat sich als besonders vorteilhaft herausgestellt, wenn das erfindungsgemäße Mittel das oder die aminofunktionalisierten Silikonpolymere (a1) in bestimmten Mengenbereichen enhält. Besonders gute Ergebnisse konnten erhalten werden, wenn das Mittel - bezogen auf das Gesamtgewicht des Mittels - in einer Gesamtmenge von 0,1 bis 8,0 Gew.-%, bevorzugt 0,2 bis 5,0 Gew.-%, weiter bevorzugt von 0,3 bis 3,0 Gew.-% und ganz besonders bevorzugt von 0,4 bis 2,5 Gew.-% enthält.

Im Rahmen einer weiteren besonders bevorzugten Ausführungsform ist ein erfindungsgemäßes Mittel dadurch gekennzeichnet, dass es - bezogen auf das Gesamtgewicht des Mittels - ein oder mehrere aminofunktionalisierte Silikonpolymere (a1) in einer Gesamtmenge von 0,1 bis 8,0 Gew.- %, bevorzugt 0,2 bis 5,0 Gew.-%, weiter bevorzugt von 0,3 bis 3,0 Gew.-% und ganz besonders bevorzugt von 0,4 bis 2,5 Gew.-% enthält.

<u>farbgebende Verbindungen (a2)</u>

Als zweiten wesentlichen Bestandteil enthält das erfindungsgemäße Mittel mindestens eine farbgebende Verbindung (a2).

Unter farbgebenden Verbindungen werden im Sinne der Erfindung Substanzen verstanden, die in der Lage sind, dem Keratinmaterial eine Färbung zu verleihen. Besonders gut geeignete farbgebende Verbindungen können ausgewählt werden aus der Gruppe der Pigmente, der direktziehenden Farbstoffe, der photochromen Farbstoffe und der thermochromen Farbstoffe.

Im Rahmen einer weiteren bevorzugten Ausführungsform ist ein erfindungsgemäßes Mittel dadurch gekennzeichnet, dass es mindestens eine farbgebende Verbindung (a2) aus der Gruppe der Pigmente, der direktziehenden Farbstoffe, der photochromen Farbstoffe und der thermochromen Farbstoffe enthält.

Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Geeignete Farbpigmente können anorganischen und/oder organischen Ursprungs sein.

In einer bevorzugten Ausführungsform ist ein erfindungsgemäßes Mittel dadurch gekennzeichnet, dass es mindestens eine farbgebende Verbindung (a2) aus der Gruppe der anorganischen und/oder organischen Pigmente enthält.

Bevorzugte Farbpigmente sind ausgewählt aus synthetischen oder natürlichen anorganischen Pigmenten. Anorganische Farbpigmente natürlichen Ursprungs können beispielsweise aus Kreide, Ocker, Umbra, Grünerde, gebranntem Terra di Siena oder Graphit hergestellt werden. Weiterhin können als anorganische Farbpigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Farbpigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Erfindungsgemäß ebenfalls besonders bevorzugte Farbpigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

In einer weiteren bevorzugten Ausführungsform ist ein erfindungsgemäßes Mittel dadurch gekennzeichnet, dass es mindestens eine farbgebende Verbindung (a2) aus der Gruppe der anorganischen Pigmente enthält, das bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

In einer weiteren bevorzugten Ausführungsform ist ein erfindungsgemäßes Mittel dadurch gekennzeichnet, dass es (a) mindestens eine farbgebende Verbindung (a2) aus der Gruppe der Pigmente enthält, die ausgewählt ist aus Pigmenten auf Mica- oder Glimmerbasis, die mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sind.

Beispiele für besonders geeignete Farbpigmente sind im Handel beispielsweise unter den Handelsbezeichnungen Rona®, Colorona®, Xirona®, Dichrona® und Timiron® von der Firma Merck, Ariabel® und Unipure® von der Firma Sensient, Prestige® von der Firma Eckart Cosmetic Colors und Sunshine® von der Firma Sunstar erhältlich.

Ganz besonders bevorzugte Farbpigmente mit der Handelsbezeichnung Colorona® sind beispielsweise:

Colorona Copper, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES)
Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491 (Iron oxides), Tin oxide

Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, CI 77891 (Titanium dioxide)
Colorona Bright Gold, Merck, Mica, CI 77891 (Titanium dioxide), CI 77491 (Iron oxides)
Colorona Blackstar Gold, Merck, MICA, CI 77499 (IRON OXIDES)

Weiterhin besonders bevorzugte Farbpigmente mit der Handelsbezeichnung Xirona® sind beispielsweise:

Xirona Golden Sky, Merck, Silica, CI 77891 (Titanium Dioxide), Tin Oxide
Xirona Caribbean Blue, Merck, Mica, CI 77891 (Titanium Dioxide), Silica, Tin Oxide
Xirona Kiwi Rose, Merck, Silica, CI 77891 (Titanium Dioxide), Tin Oxide
Xirona Magic Mauve, Merck, Silica, CI 77891 (Titanium Dioxide), Tin Oxide.

Zudem sind besonders bevorzugte Farbpigmente mit der Handelsbezeichnung Unipure® beispielsweise:

Unipure Red LC 381 EM, Sensient CI 77491 (Iron Oxides), Silica
Unipure Black LC 989 EM, Sensient, CI 77499 (Iron Oxides), Silica
Unipure Yellow LC 182 EM, Sensient, CI 77492 (Iron Oxides), Silica

Im Rahmen einer weiteren Ausführungsform kann das erfindungsgemäße Mittel auch ein oder mehrere farbgebende Verbindungen (a2) aus der Gruppe der organischen Pigmente enthalten

Bei den erfindungsgemäßen organischen Pigmenten handelt es sich um entsprechend unlösliche, organische Farbstoffe oder Farblacke, die beispielsweise aus der Gruppe der Nitroso-, Nitro- Azo-, Xanthen-, Anthrachinon-, Isoindolinon-, Isoindolin-, Chinacridon-, Perinon-, Perylen- , Diketopyrrolopyorrol-, Indigo-, Thioindido-, Dioxazin-, und/oder Triarylmethan-Verbindungen ausgewählt sein können.

Als besonders gut geeignete organische Pigmente können beispielsweise Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470 genannt werden.

In einer weiteren besonders bevorzugten Ausführungsform ist ein erfindungsgemäßes Mittel dadurch gekennzeichnet, dass es mindestens eine farbgebende Verbindungen (a2) aus der Gruppe der organischen Pigmente enthält, die bevorzugt ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Aufgrund ihrer ausgezeichneten Licht- und Temperaturbeständigkeit ist die Verwendung der zuvor genannten Pigmente in dem Mittel ganz besonders bevorzugt. Weiterhin ist es bevorzugt, wenn die eingesetzten Pigmente eine bestimmte Teilchengröße aufweisen. Es ist daher erfindungsgemäß vorteilhaft, wenn das mindestens eine Pigment eine mittlere Teilchengröße $D_{50}$ von 1,0 bis 50 $\mu$m, vorzugsweise von 5,0 bis 45 $\mu$m, bevorzugt von 10 bis 40 $\mu$m, insbesondere von 14 bis 30 $\mu$m, aufweist. Die mittlere Teilchengröße $D_{50}$ kann beispielsweise unter Verwendung von dynamischer Lichtstreuung (DLS) bestimmt werden.

Die farbgebenden Verbindungen (a2), inbesondere die farbgebenden Verbindungen aus der Gruppe der Pigmente, stellen den zweiten wesentlichen des erfindungsgemäßen Mittels dar und werden bevorzugt in bestimmten Mengenbereichen im Mittel eingesetzt. Besonders gute Ergebnisse wurden erhalten, wenn das Mittel - bezogen auf das Gesamtgewicht des Mittels - ein odere mehrere Pigmente (a2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5

Gew.-% enthielt.

In einer weiteren ganz besonders bevorzugten Ausführungsform ist ein erfindungsgemäßes Mittel dadurch gekennzeichnet, dass das Mittel - bezogen auf das Gesamtgewicht des Mittels - ein odere mehrere Pigmente (a2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthält.

Als farbgebende Verbindungen (a2) können die erfindungsgemäßen Mittel auch einen oder mehrere direktziehende Farbstoffe enthalten. Bei direktziehenden Farbstoffen handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone, Triarylmethanfarbstoffe oder Indophenole.

Die direktziehenden Farbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die direktziehenden Farbstoffe im Sinne der vorliegenden Erfindung eine Löslichkeit in Wasser (760 mmHg) bei 25 °C von mehr als 1,0 g/L.

Direktziehende Farbstoffe können in anionische, kationische und nichtionische direktziehende Farbstoffe unterteilt werden.

In einer weiteren Ausführungsform ist ein erfindungsgemäßes Verfahren dadurch gekennzeichnet, dass das Mittel mindestens eine farbgebende Verbindung (a2) aus der Gruppe der anionischen, nichtionischen und kationischen direktziehenden Farbstoffe enthält.

Geeignete kationische direktziehende Farbstoffe sind beispielsweise Basic Blue 7, Basic Blue 26, HC Blue 16, Basic Violet 2 und Basic Violet 14, Basic Yellow 57, Basic Red 76, Basic Blue 16, Basic Blue 347 (Cationic Blue 347 / Dystar), HC Blue No. 16, Basic Blue 99, Basic Brown 16, Basic Brown 17, Basic Yellow 57, Basic Yellow 87, Basic Orange 31, Basic Red 51 Basic Red 76.

Als nichtionische direktziehende Farbstoffe können beipsielsweise nichtionische Nitro- und Chinonfarbstoffe und neutrale Azofarbstoffe eingesetzt werden. Geeigente nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Anionische direktziehende Farbstoffe werden auch als Säurefarbstoffe bezeichnet. Unter Säurefarbstoffen werden direktziehende Farbstoffe verstanden, die mindestens eine Carbonsäuregruppierung (-COOH) und/oder eine Sulfonsäuregruppierung ($-SO_3H$) besitzen. In Abhängigkeit vom pH-Wert liegen die protonierten Formen (-COOH, $-SO_3H$) der Carbonsäure- bzw. Sulfonsäuregruppierungen mit ihren deprotonierten Formen ($-COO^-$, $-SO_3^-$ vor) im Gleichgewicht vor. Mit abnehmendem pH-Wert steigt der Anteil der protonierten Formen. Werden direktziehende Farbstoffe in Form ihrer Salze eingesetzt, so liegen die Carbonsäuregruppen bzw. Sulfonsäuregruppen in deprotonierter Form vor und sind zur Einhaltung der Elektroneutralität mit entsprechenden stöchiometrischen Äquivalente an Kationen neutralisiert. Erfindungsgemäße Säurefarbstoffe können auch in Form ihrer Natriumsalze und/oder ihrer Kaliumsalze eingesetzt werden.

Die Säurefarbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die Säurefarbstoffe im Sinne der vorliegenden Erfindung besitzen eine Löslichkeit in Wasser (760 mmHg) bei 25 °C von mehr als 1,0 g/L.

Die Erdalkalisalze (wie beispielsweise Calciumsalze und Magnesiumsalze) bzw. Aluminiumsalze von Säurefarbstoffen besitzen oftmals eine schlechtere Löslichkeit als die entsprechenden Alkalisalze. Sofern die Löslichkeit dieser Salze unterhalb von 0,5 g/L (25 °C, 760 mmHg) liegt, fallen diese nicht unter die Definition eines direktziehenden Farbstoffes.

Ein wesentliches Merkmal der Säurefarbstoffe ist ihr Vermögen, anionische Ladungen auszubilden, wobei die hierfür verantwortlichen Carbonsäure- bzw. Sulfonsäuregruppen üblicherweise an verschiedene chromophore Systeme geknüpft sind. Geeignete chromophore Systeme finden sich beispielsweise in den Strukturen von Nitrophenylendiaminen, Nitroaminophenolen, Azofarbstoffen, Anthrachinonfarbstoffen, Triarylmethanfarbstoffen, Xanthen-Farbstoffen, Rhodamin-Farbstoffen, Oxazinfarbstoffen und/oder Indophenolfarbstoffen.

Im Rahmen einer weiteren Ausführungsform ist ein Verfahren zum Färben von keratinischem Material dadurch gekennzeichnet ist, dass das Mittel mindestens einen anionischen direktziehenden Farbstoff enthält, der ausgewählt ist aus der Gruppe der Nitrophenylendiamine, der Nitroaminophenole, der Azofarbstoffe, der Anthrachinonfarbstoffe, der

Triarylmethanfarbstoffe, der Xanthen-Farbstoffe, der Rhodamin-Farbstoffe, der Oxazinfarbstoffen und/oder der Indophenolfarbstoffe, wobei die Farbstoffe aus der vorgenannten Gruppe jeweils mindestens eine Carbonsäuregruppe (-COOH), eine Natriumcarboxylatgruppe (-COONa), eine Kaliumcarboxylatgruppe (-COOK), eine Sulfonsäuregruppe ($-SO_3H$) eine Natriumsulfonatgruppe ($-SO_3Na$) und/oder eine Kaliumsulfonatgruppe ($-SO_3K$) besitzen.

Als geeignete Säurefarbstoffe können beispielsweise eine oder mehrere Verbindungen aus der folgenden Gruppe ausgewählt werden: Acid Yellow 1 (D&C Yellow 7, Citronin A, Ext. D&C Yellow No. 7, Japan Yellow 403,CI 10316, COLIPA n° B001), Acid Yellow 3 (COLIPA n° : C 54, D&C Yellow N° 10, Quinoline Yellow, E104, Food Yellow 13), Acid Yellow 9 (CI 13015), Acid Yellow 17 (CI 18965), Acid Yellow 23 (COLIPA n° C 29, Covacap Jaune W 1100 (LCW), Sicovit Tartrazine 85 E 102 (BASF), Tartrazine, Food Yellow 4, Japan Yellow 4, FD&C Yellow No. 5), Acid Yellow 36 (CI 13065), Acid Yellow 121 (CI 18690), Acid Orange 6 (CI 14270), Acid Orange 7 (2-Naphthol orange, Orange II, CI 15510, D&C Orange 4, COLIPA n° C015), Acid Orange 10 (C.I. 16230; Orange G sodium salt), Acid Orange 11 (CI 45370), Acid Orange 15 (CI 50120), Acid Orange 20 (CI 14600), Acid Orange 24 (BROWN 1;CI 20170;KATSU201;nosodiumsalt;Brown No.201;RESORCIN BROWN;ACID ORANGE 24;Japan Brown 201;D & C Brown No.1), Acid Red 14 (C.I.14720), Acid Red 18 (E124, Red 18; CI 16255), Acid Red 27 (E 123, CI 16185, C-Rot 46, Echtrot D, FD&C Red Nr.2, Food Red 9, Naphtholrot S), Acid Red 33 (Red 33, Fuchsia Red, D&C Red 33, CI 17200), Acid Red 35 (CI C.I.18065), Acid Red 51 (CI 45430, Pyrosin B, Tetraiodfluorescein, Eosin J, Iodeosin), Acid Red 52 (CI 45100, Food Red 106, Solar Rhodamine B, Acid Rhodamine B, Red n° 106 Pontacyl Brilliant Pink), Acid Red 73 (CI CI 27290), Acid Red 87 (Eosin, CI 45380), Acid Red 92 (COLIPA n° C53, CI 45410), Acid Red 95 (CI 45425, Erythtosine,Simacid Erythrosine Y), Acid Red 184 (CI 15685), Acid Red 195, Acid Violet 43 (Jarocol Violet 43, Ext. D&C Violet n° 2, C.I. 60730, COLIPA n° C063), Acid Violet 49 (CI 42640), Acid Violet 50 (CI 50325), Acid Blue 1 (Patent Blue, CI 42045), Acid Blue 3 (Patent Blau V, CI 42051), Acid Blue 7 (CI 42080), Acid Blue 104 (CI 42735), Acid Blue 9 (E 133, Patentblau AE, Amidoblau AE, Erioglaucin A, CI 42090, C.I. Food Blue 2), Acid Blue 62 (CI 62045), Acid Blue 74 (E 132, CI 73015), Acid Blue 80 (CI 61585), Acid Green 3 (CI 42085, Foodgreen1), Acid Green 5 (CI 42095), Acid Green 9 (C.I.42100), Acid Green 22 (C.I.42170), Acid Green 25 (CI 61570, Japan Green 201, D&C Green No. 5), Acid Green 50 (Brillantsäuregrün BS, C.I. 44090, Acid Brilliant Green BS, E 142), Acid Black 1 (Black n° 401, Naphthalene Black 10B, Amido Black 10B, CI 20 470, COLIPA n° B15), Acid Black 52 (CI 15711), Food Yellow 8 (CI 14270), Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Die Wasserlöslichkeit der anionischen direktziehenden Farbstoffe kann beispielsweise auf dem folgenden Weg bestimmt werden. 0,1 g des anionischen direktziehenden Farbstoffes werden in ein Becherglas gegeben. Es wird ein Rührfisch hinzugegeben. Dann werden 100 ml Wasser hinzugefügt. Diese Mischung wird auf einem Magnetrührer unter Rühren auf 25 °C erwärmt. Es wird für 60 Minuten gerührt. Danach wird die wässrige Mischung visuell beurteilt. Sind noch ungelöste Reste vorhanden, wird die Wassermenge - beispielsweise in Schritten von 10 ml - erhöht. Es wird solange Wasser zugegeben, bis sich die eingesetzte Farbstoffmenge komplett gelöst hat. Sofern sich die Farbstoff-Wasser-Mischung aufgrund der hohen Intenstität des Farbstoffes nicht visuell beurteilten lässt, wird die Mischung filtriert. Bleibt auf dem Filterpapier ein Anteil an ungelösten Farbstoffen zurück, wird der Löslichkeitsversuch mit einer höheren Wassermenge wiederholt. Lösen sich 0,1 g des anionischen direktziehenden Farbstoffes bei 25 °C in 100 ml Wasser, so liegt die Löslichkeit des Farbstoffes bei 1,0 g/L.

Acid Yellow 1 trägt den Namen 8-Hydroxy-5,7-dinitro-2-naphthalenesulfonsäure Dinatriumsalz und besitzt eine Löslichkeit in Wasser von mindestens 40 g/L (25°C).

Acid Yellow 3 ist ein Gemisch der Natriuimsalze von Mono- und Sisulfonsäuren von 2-(2-Chinolyl)-1H-indene-1,3(2H)-dion und besitzt eine Wasserlöslichkeit von 20 g/L (25 °C).

Acid Yellow 9 ist das Dinatriumsalz der 8-Hydroxy-5,7-dinitro-2-naphthalenesulfonsäure, seine Wasserlöslichkeit liegt oberhalb von 40 g/L (25 °C).

Acid Yellow 23 ist das Trinatriumsalz der 4,5-Dihydro-5-oxo-1-(4-sulfophenyl)-4-((4-sulfophenyl)azo)-1H-pyrazole-3-carbonsäure und bei 25 °C gut in Wasser löslich.

Acid Orange 7 ist das Natriumsalz des 4-[(2-Hydroxy-1-naphthyl)azo]benzensulfonats. Seine Wasserlöslichkeit beträgt mehr als 7 g/L (25 °C).

Acid Red 18 ist das Trinatirumsalz von 7-Hydroxy-8-[(E)-(4-sulfonato-1-naphthyl)-diazenyl)]-1,3-naphthalenedisulfonat und besitzt eine sehr hohe Wasserlöslichkeit von mehr als 20 GEw.-%. Acid Red 33 ist das Diantriumsalz des 5-Amino-4-hydroxy-3-(phenylazo)-naphthalene-2,7-disulphonats, seine Wasserlöslichkeit liegt bei 2,5 g/L (25 °C).

Bei Acid Red 92 handelt es sich um das Dinatriumsalz der 3,4,5,6-Tetrachloro-2-(1 ,4,5,8-tetrabromo-6-hydroxy-3-oxoxanthen-9-yl)benzoesäure, dessen Wasserlöslichkeit mit größer 10 g/L angegeben wird (25 °C).

Acid Blue 9 ist das Dinatriumsalz von 2-({4-[N-ethyl(3-sulfonatobenzyl]amino]phenyl}{4-[(N-ethyl(3-sulfonatobenzyl)imino]-2,5-cyclohexadien-1-ylidene}methyl)-benzenesulfonat und besitzt eine Wasserlöslichkeit von mehr als 20 Gew.-% (25 °C).

In einer weiteren Ausführungsform ist ein erfindungsgemäßes Mittel daher dadurch gekennzeichnet, dass es mindestens einen direktziehenden Farbstoff (a2) enthält, der ausgewählt ist aus der Gruppe aus Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid

Orange 10, Acid Orange 11, Acid Orange 15, Acid Orange 20, Acid Orange 24, Acid Red 14, Acid Red, Acid Red 27, Acid Red 33, Acid Red 35, Acid Red 51, Acid Red 52, Acid Red 73, Acid Red 87, Acid Red 92, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Der oder die direktziehenden Farbstoffe können je nach erwünschter Farbintensität in verschiedenen Mengen im Mittel eingesetzt werden. Gute Ergebnisse konnten erhalten werden, wenn das Mittel - bezogen auf das Gesamtgewicht des Mittels - ein oder mehrere direktziehende Farbstoffe (a2) in einer Gesamtmenge von 0,01 bis 10,0 Gew.-%, bevorzugt von 0,1 bis 8,0 Gew.-%, weiter bevorzugt von 0,2 bis 6,0 Gew.-% und ganz besonders bevorzugt von 0,5 bis 4,5 Gew.-% enthält.

Weiterhin kann das Mittel als farbgebende Verbindung (a2) auch mindestens einen photochromen oder thermochromen Farbstoff enthalten.

Photochrome Farbstoffe sind Farbstoffe, die auf Bestrahlung mit UV-Licht (Sonnenlicht oder Schwarzlicht) mit einer reversiblen Farbtonänderung reagieren. Dabei verändert das UV-Licht die chemische Struktur der Farbstoffe und damit ihr Absorptionsverhalten (Photochromie).

Thermochrome Farbstoffe sind Farbstoffe, die auf Temperaturänderungen mit einer reversiblen Farbtonänderung reagieren. Dabei verändert die Temperaturänderung die chemische Struktur der Farbstoffe und damit ihr Absorptionsverhalten (Thermochromie).

Das Mittel kann - bezogen auf das Gesamtgewicht des Mittels - ein oder mehrere photochrome Farbstoffe (a2) in einer Gesamtmenge von 0,01 bis 10,0 Gew.-%, bevorzugt von 0,1 bis 8,0 Gew.- %, weiter bevorzugt von 0,2 bis 6,0 Gew.-% und ganz besonders bevorzugt von 0,5 bis 4,5 Gew.-% enthalten

<u>Anlagerungsprodukte von $C_1$-$C_6$-Alkylenoxid(en) an die Ester aus $C_{12}$-$C_{30}$-Fettsäuren und aromatischen $C_1$-$C_{12}$-Alkoholen (a3)</u>

Als dritten wesentlichen Inhaltsstoff (a3) enthalten die erfindungsgemäßen Mittel mindestens ein Anlagerungsprodukt von $C_1$-$C_6$-Alkylenoxid(en) an die Ester aus $C_{12}$-$C_{30}$-Fettsäuren und aromatischen $C_1$-$C_{12}$-Alkoholen.

Die Anlagerungsprodukte von $C_1$-$C_6$-Alkylenoxid(en) an die Ester aus $C_{12}$-$C_{30}$-Fettsäuren und aromatischen $C_1$-$C_{12}$-Alkoholen (a3) werden im Folgenden vekürzt auch als alkoxylierte Fettsäureester (a3) bezeichnet.

Bei $C_1$-$C_6$-Alkylenoxiden handelt es sich um die Epoxide von $C_1$-$C_6$-Alkanen. Als besonders gut geeignete $C_1$-$C_6$-Alkylenoxide können beispielsweise Ethylenoxid (1,2-Epoxyethan), Propylenoxid (1,2-Epoxypropan) und Butylenoxide (1,2-Epoxybutan sowie 2,3-Epoxybutan) genannt werden.

Die ethoxylierten Fettsäure-Ester (a3) basieren auf $C_{12}$-$C_{30}$-Fettsäuren. Bei diesen erfindungsgemäßen $C_{12}$-$C_{30}$-Fettsäuren handelt es sich um lineare oder verzweigte, gesättigte oder einfach oder mehrfach ungesättigte Fettsäuren, die auch eine oder mehrere Hydroxygruppen tragen können. Die erfindungsgemäßen $C_{12}$-$C_{24}$-Fettsäuren sind dadurch gekennzeichnet, dass die 12 bis 30 Kohlenstoffatome, bevorzugt 12 bis 24 Kohlenstoffatome, umfassen. Weiterhin tragen die $C_{12}$-$C_{24}$-Fettsäuren mindestens eine Carbonsäure-Gruppierung.

Zur Ausbildung der erfindungsgemäßen ethoxylierten Fettsäureester (a3) können beispielsweise eine oder mehrere Fettsäuren eingesetzt werden, die ausgewählt sind aus der Gruppe aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure], Nervonsäure [(15Z)-Tetracos-15-ensäure] und/oder Rizinusölsäure ((9Z,12R)-12-Hydroxy-9-octadecensaeure.

Die ethoxylierten Fettsäure-Ester (a3) stellen Anlagerungsprodukte von $C_1$-$C_6$-Alkylenoxid(en) an die Ester der zuvor beschriebenen $C_{12}$-$C_{24}$-Fettsäuren und aromatischen $C_1$-$C_{12}$-Alkoholen dar. Kennzeichnend für die aromatischen $C_1$-$C_{12}$-Alkohole ist, dass sie 1 bis 12 Kohlenstoffatome und mindestens ein aromatisches Ringsystem umfassen. Die aromatische $C_1$-$C_{12}$-Alkohole besitzen jeweils mindestens eine Hydroxy-Gruppe, die sich entweder direkt am Aromaten befinden kann (wie z.B. bei Phenol) oder aber über eine aliphatische Einheit mit dem Aromaten verknüpft sein kann (wie z.B. bei Benzylalkohol oder 2-Phenoxyethanol). Die Strukturen der aromatischen $C_1$-$C_{12}$-Alkohole können auch noch weitere Heteroatome wie Sauerstoff oder Stickstoff umfassen.

Die entsprechenden aromatischen $C_1$-$C_{12}$-Alkohole können ein- oder mehrwertige Alkohole sein, d.h. die Alkohole können eine oder mehrere Hydroxygruppen besitzen.

Einwertige aromatische $C_1$-$C_{12}$-Alkohole sind ganz besonders bevorzugt. Diese Verbindungsklasse umfasst genau eine Hydroxygruppe. Als geeignete Vertreter können beispielsweise Phenol, Benzylalkohol, 2-Phenylethylalkohol

und 2-Phenoxyethanol genannt werden.

Besonders bevorzugt ist demzufolge ein Mittel zum Färben von keratinischem Material, insbesondere menschlichen Haaren, enthaltend

(a1) mindestens ein aminofunktionalisiertes Silikonpolymer, und
(a2) mindestens eine farbgebende Verbindung,
(a3) mindestens ein Anlagerungsprodukt von $C_1$-$C_6$-Alkylenoxid(en) an die Ester aus $C_{12}$-$C_{30}$-Fettsäuren und einwertigen aromatischen $C_1$-$C_{12}$-Alkoholen, und
(a4) mindestens ein Anlagerungsprodukt von C1 -C6 -Alkylenoxid(en) an aliphatische C1-C24 -Alkanole der Formel (AA-I).

Besonders gute Waschechtheiten und Reibechtheiten konnten erhalten werden, wenn im erfindungsgemäßen Mittel mindestens ein Anlagerungsprodukt (a3) von $C_1$-$C_6$-Alkylenoxid(en) an einen Ester eingesetzt wurde, der durch Veresterung von einer $C_{12}$-$C_{30}$-Fettsäure mit einem aromatischen $C_1$-$C_{12}$-Alkohol aus der Gruppe aus Benzylalkohol, Phenol, 2-Phenylethylalkohol und 2-Phenoxyethanol, erhalten wird.

Im Rahmen einer weiteren bevorzugten Ausführungsform ist ein erfindungsgemäßes Mittel dadurch gekennzeichnet, dass es
(a3) mindestens ein Anlagerungsprodukt von $C_1$-$C_6$-Alkylenoxid(en) an einen Ester enthält, der durch Veresterung von einer $C_{12}$-$C_{24}$-Fettsäure mit einem aromatischen $C_1$-$C_{12}$-Alkohol aus der Gruppe aus Benzylalkohol, Phenol, 2-Phenylethylalkohol und 2-Phenoxyethanol, ganz besonders bevorzugt Benzylalkohol, erhalten wird.

Im Rahmen einer besonders bevorzugten Ausführungsform ist ein erfindungsgemäßes Mittel dadurch gekennzeichnet, dass es
(a3) mindestens ein Anlagerungsprodukt von $C_1$-$C_6$-Alkylenoxid(en) an einen Ester enthält, der durch Veresterung von einer $C_{12}$-$C_{24}$-Fettsäure mit Benzylalkohol erhalten wird.

Im Rahmen einer weiteren bevorzugten Ausführungsform ist ein erfindungsgemäßes Mittel dadurch gekennzeichnet, dass es
(a3) mindestens ein Anlagerungsprodukt von Ethylenoxid und/oder Propylenoxid an einen Ester enthält, der durch Veresterung von einer $C_{12}$-$C_{24}$-Fettsäure mit einem aromatischen $C_1$-$C_{12}$-Alkohol aus der Gruppe aus Benzylalkohol, Phenol, 2-Phenylethylalkohol und 2-Phenoxyethanol erhalten wird.

Im Rahmen einer weiteren besonder bevorzugten Ausführungsform ist ein erfindungsgemäßes Mittel dadurch gekennzeichnet, dass es
(a3) mindestens ein Anlagerungsprodukt von Ethylenoxid und/oder Propylenoxid an einen Ester enthält, der durch Veresterung von einer $C_{12}$-$C_{24}$-Fettsäure mit Benzylalkohol erhalten wird.

Wie bereits zuvor beschrieben können als besonders gut geeignete $C_1$-$C_6$-Alkylenoxide beispielsweise Ethylenoxid (1,2-Epoxyethan), das Propylenoxid (1,2-Epoxypropan) und Butylenoxide (1,2-Epoxybutan sowie 2,3-Epoxybutan) eingesetzt werden. Ethylenoxid (1,2-Epoxyethan) und Propylenoxid (1,2-Epoxypropan) sind explizit ganz besonders bevorzugt. Am allermeisten bevorzugt ist Propylenoxid (1,2-Epoxypropan).

Ein entsprechendes Anlagerungsprodukt von Ethylenoxid an die Ester aus $C_{12}$-$C_{30}$-Fettsäuren und aromatischen $C_1$-$C_{12}$-Alkoholen (a3) entsteht, wenn die $C_{12}$-$C_{30}$-Fettsäure selbst oder der bereits aus ihr ausgebildete Ester mit Ethylenoxid (Alternativanme 1,2-Epoxyethan, CAS-Nummer 75-21-8) umgesetzt wird.

Analog entsteht ein entsprechendes Anlagerungsprodukt von Propylenoxid an die Ester aus $C_{12}$-$C_{30}$-Fettsäuren und aromatischen $C_1$-$C_{12}$-Alkoholen (a3), wenn die $C_{12}$-$C_{30}$-Fettsäure selbst oder der bereits aus ihr ausgebildete Ester mit Propylenoxid (Alternativname 1,2-Epoxypropan, CAS-Nummern 75-56-9 (Racemat), 15448-47-2 ((R)-Enantiomer, 16088-62-3 (S)-Enantiomer) umgesetzt wird.

Wird die $C_{12}$-$C_{30}$-Fettsäure selbst mit Ethylenoxid umgesetzt, so kann sich zunächst ein Addukt ausgehend von der Carbonsäure-Gruppierung der $C_{12}$-$C_{30}$-Fettsäure und dem Ethylenoxid ausbilden, so dass eine Gruppierung *-C(O)-O-$CH_2$-$CH_2$-O-* entsteht. Bei dieser Gruppierung handelt es sich ebenfalls um einen Ester. Wird pro Mol Fettsäure ein Mol Ethylenoxid umgesetzt, so bildet sich im Mittel ein einfaches Addukt mit einer Einheit *-CH2-CH2-O-* aus. Abhängig davon, in welchem molaren Überschuss das Ethylenoxid eingesetzt wird, können sich jedoch auch mehrfache Addukte ausbilden, wobei pro Mol $C_{12}$-$C_{30}$-Fettsäure mehrere Einheiten *-CH2-CH2-O-* vorliegen. Zur Ausbildung des Esters (a3) wird dieses Addukt dann weiterhin mit mindestens einem aromatischen $C_1$-$C_{12}$-Alkohol umgesetzt. Die mit einem Stern gekennzeichneten Positionen stellen hierbei die Bindung zum restlichen Teil der Fettsäure und die Bindung mit dem restlichen Teil des Alkohols dar.

Wird die $C_{12}$-$C_{30}$-Fettsäure analog mit Propylenoxid umgesetzt, so kann sich zunächst ein Addukt ausgehend von der Carbonsäure-Gruppierung der $C_{12}$-$C_{30}$-Fettsäure und dem Propylenoxid ausbilden, so dass eine Gruppierung *-C(O)-O-CH($CH_3$)-$CH_2$-O-* oder eine Gruppierung *-C(O)-O-$CH_2$-CH($CH_3$)-O-*. Im Reaktionsgemisch wird üblicherweise eine Mischung der beiden vorgenannten Gruppierungen erhalten. Bei beiden Gruppierungen handelt es sich ebenfalls um Ester. Wird pro Mol Fettsäure ein Mol Propylenoxid umgesetzt, so bildet sich im Mittel ein einfaches Addukt

mit einem Gemisch der Einheiten *-$CH(CH_3)$-$CH_2$-O-* und *-$CH_2$-$CH(CH_3)$-O-* aus. Abhängig davon, in welchem molaren Überschuss das Propylenoxid eingesetzt wird, können sich jedoch auch mehrfache Addukte ausbilden, wobei pro Mol $C_{12}$-$C_{30}$-Fettsäure dann mehrere Einheiten *-$CH(CH_3)$-$CH_2$-O-* und/oder *-$CH_2$-$CH(CH_3)$-O-* vorliegen. Zur Ausbildung des Esters (a3) wird dieses Addukt dann weiterhin mit mindestens einem aromatischen $C_1$-$C_{12}$-Alkohol umgesetzt. Die mit einem Stern gekennzeichneten Positionen stellen hierbei die Bindung zum restlichen Teil der Fettsäure und die Bindung mit dem restlichen Teil des Alkohols dar.

Weiterhin ist es prinzipiell denkbar, dass die $C_{12}$-$C_{30}$-Fettsäure mit einer Mischung aus Ethylenoxid und Propylenoxid umgesetzt wird. In diesem Fall bilden sich Gemische der zuvor beschriebenen Addukte aus. Auch die Umsetzungen von höheren Alkylenoxiden wie beispielsweise Butylenoxiden sind auf diese Weise mit den $C_{12}$-$C_{30}$-Fettsäuren möglich.

Die auf diese Weise hergestellten Anlagerungsprodukte führen beispielsweise zu den alkoxylierten Fettsäureestern der allgemeinen Formel (AFE-I)

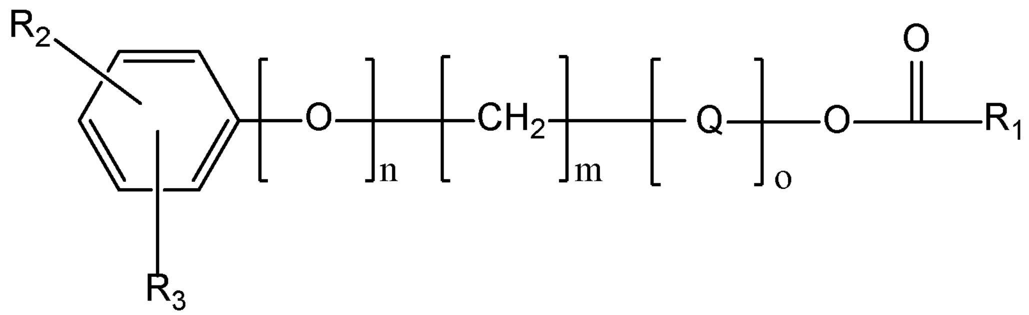

(AFE-I)

wobei

| | |
|---|---|
| R1 | für eine gesättigte oder ungesättige $C_{11}$-$C_{29}$-Alkylgruppe steht |
| R2, R3 | unabhängig voneinander für ein Wasserstoffatom, eine $C_1$-$C_6$-Alkylgruppe, eine Hydroxygruppe oder eine $C_1$-$C_6$-Alkoxygruppe stehen, |
| n | für die Zahl 0 oder 1 steht, |
| m | für eine ganze Zahl von 0 bis 6 steht, |
| o | für eine ganze Zahl von 1 bis 60 steht, und |
| Q | für eine Struktureinheit -O-$CH_2$-$CH_2$- , -O-$CH(CH_3)$-$CH_2$- oder -O-$CH_2$-$CH(CH_3)$- steht, |

wobei die Maßgabe besteht, dass, wenn m gleich 0 ist, auch n gleich 0 ist.

Mit Mitteln, die mindestens ethoxylierten Fettsäureester (a3) der Formel (AFE-I) enthielten, wurden Färbungen erhalten, die sich im Hinblick auf gute Waschechtheiten und gute Reibechtheiten besonders auszeichneten. Aus diesem Grund ist der Einsatz einer oder mehrerer ethoxylierter Fettsäureester (a3) der Formel (AFE-I) in den erfindungsgemäßen Mitteln ganz besonders bevorzugt.

Im Rahmen einer weiteren besonders bevorzugten Ausführungsform ist ein erfindungsgemäßes Mittel dadurch gekennzeichnet, dass es

(a3) mindestens einen alkoxylierten Fettsäureester der allgemeinen Formel (AFE-I) enthält

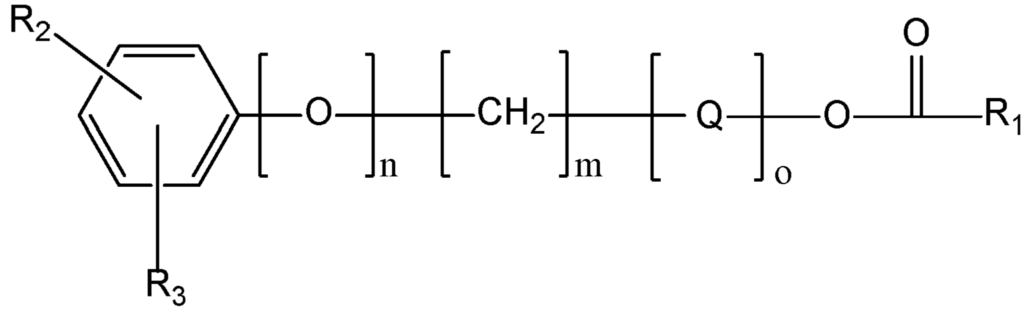

(AFE-I)

wobei

R1 für eine gesättigte oder ungesättigte $C_{11}$-$C_{29}$-Alkylgruppe steht
R2, R3 unabhängig voneinander für ein Wasserstoffatom, eine $C_1$-$C_6$-Alkylgruppe, eine Hydroxygruppe oder eine $C_1$-$C_6$-Alkoxygruppe stehen,
n für die Zahl 0 oder 1 steht,
m Q für eine ganze Zahl von 0 bis 6 steht,
o für eine ganze Zahl von 1 bis 60 steht, und für eine Struktureinheit $-O-CH_2-CH_2-$ , $-O-CH(CH_3)-CH_2-$ oder $-O-CH_2-CH(CH_3)-$ steht,

wobei die Maßgabe besteht, dass, wenn m gleich 0 ist, auch n gleich 0 ist.

Der Rest R1 steht für eine gesättigte oder ungesättigte $C_{11}$-$C_{29}$-Alkylgruppe. Eine ungesättigte $C_{11}$-$C_{23}$-Alkylgruppe kann eine oder mehrere Doppelbindungen umfassen und wird alternativ auch als ungesättigte $C_{11}$-$C_{23}$-Alkenylgruppe bezeichnet. Die gesättigte oder ungesättigte $C_{11}$-$C_{29}$-Alkylgruppe kann linerar oder verzweigt sein.

Bevorzugt steht R1 für eine lineare, gesättigte oder ungesättigte $C_{11}$-$C_{23}$-Alkylgruppe.

Die Reste R2 und R3 stehen voneinander für ein Wasserstoffatom, eine $C_1$-$C_6$-Alkylgruppe, eine Hydroxygruppe oder eine $C_1$-$C_6$-Alkoxygruppe. Ganz besonders bevorzugt stehen die Reste R2 und R3 beide für ein Wasserstoffatom.

Die Index-Zahl n steht für die Zahl 0 oder 1. Bevorzugt steht n für die Zahl 0.

Die Index-Zahl m steht für eine ganze Zahl von 0 bis 6. Bevorzugt steht m für die Zahl 1.

Die Index-Zahl o steht für eine ganze Zahl von 1 bis 60. Bevorzugt steht o für eine ganze Zahl von 1 bis 30, weiter bevorzugt von 1 bis 20, noch weiter bevorzugt von 1 bis 10 und ganz besonders bevorzugt von 1 bis 5.

Der Rest Q steht für eine Struktureinheit $-O-CH_2-CH_2-$ , $-O-CH(CH_3)-CH_2-$ oder $-O-CH_2-CH(CH_3)-$steht. Besonders bevorzugt steht Q für eine Struktureinheit $-O-CH(CH_3)-CH_2-$ oder $-O-CH_2-CH(CH_3)-$.

Wenn o für eine Zahl von größer als 1 steht, sind in den Verbindungen der Formel (AFE-I) (bzw. auch der Formel (AFE-II) mehrere Struktureinheiten Q enthalten, in diesem Fall kann jede Struktureinheit Q von den anderen Struktureinheiten Q unabhängig gewählt werden.

Demzufolge ist ein bevorzugtes erfindungsgemäßes Mittel dadurch gekennzeichnet, dass es
(a3) mindestens einen alkoxylierten Fettsäureester der allgemeinen Formel (AFE-I) enthält

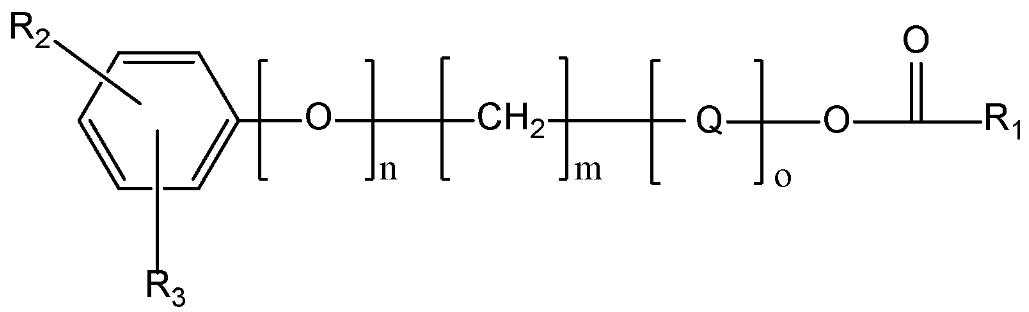

(AFE-I)

wobei

R1 für eine gesättigte oder ungesättigte $C_{11}$-$C_{29}$-Alkylgruppe steht
R2, R3 unabhängig voneinander für ein Wasserstoffatom, eine $C_1$-$C_6$-Alkylgruppe, eine Hydroxygruppe oder eine $C_1$-$C_6$-Alkoxygruppe stehen,
n für die Zahl 0 oder 1 steht,
m für eine ganze Zahl von 0 bis 6 steht,
o für eine ganze Zahl von 1 bis 60 steht, und
Q für eine Struktureinheit $-O-CH_2-CH_2-$ , $-O-CH(CH_3)-CH_2-$ oder $-O-CH_2-CH(CH_3)-$ steht,

wobei die Maßgabe besteht, dass, wenn m gleich 0 ist, auch n gleich 0 ist,
und wobei im Fall von o größer 1 jede Struktureinheit Q unabhängig von den anderen Struktureinheiten Q gewählt werden kann.

Zusammenfassend wurden mit den erfindungsgemäßen Mitteln besonders gute Ergebnisse erhalten,welche (a3) mindestens einen alkoxylierten Fettsäureester der allgemeinen Formel (AFE-I) enthielten,

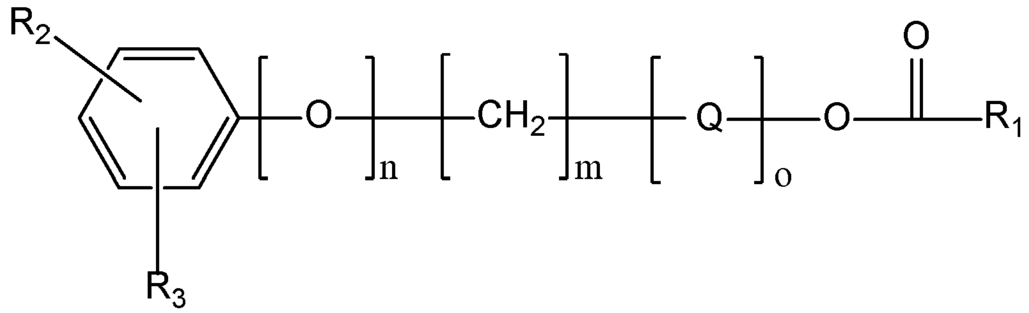

(AFE-I)

wobei

R1 für eine gesättigte oder ungesättige $C_{11}$-$C_{29}$-Alkylgruppe steht
R2, R3 beide für ein Wasserstoffatom stehen,
n für die Zahl 0 steht,
m für die Zahl 1 steht,
o für eine ganze Zahl von 1 bis 30, bevorzugt von 1 bis 20, weiter bevorzugt von 1 bis 10 und ganz besonders bevorzugt von 1 bis 5 steht, und
Q für eine Struktureinheit -O-CH($CH_3$)-$CH_2$- oder -O-$CH_2$-CH($CH_3$)- steht.

Im Rahmen einer weiteren ganz besonders bevorzugten Ausführungsform ist ein erfindungsgemäßes Mittel dadurch gekennzeichnet, dass es
(a3) mindestens einen alkoxylierten Fettsäureester der allgemeinen Formel (AFE-I) enthält

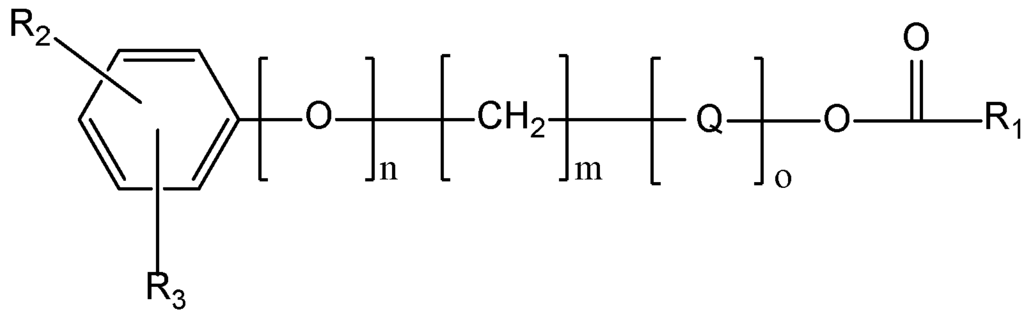

(AFE-I)

wobei

R1 für eine gesättigte oder ungesättige $C_{11}$-$C_{29}$-Alkylgruppe steht
R2, R3 beide für ein Wasserstoffatom stehen,
n für die Zahl 0 steht,
m für die Zahl 1 steht,
o für eine ganze Zahl von 1 bis 30, bevorzugt von 1 bis 20, weiter bevorzugt von 1 bis 10 und ganz besonders bevorzugt von 1 bis 5 steht, und
Q für eine Struktureinheit -O-CH($CH_3$)-$CH_2$- oder -O-$CH_2$-CH($CH_3$)- steht.

Die ganz besonders bevorzugten alkoxylierten Fettsäureester dieser Ausführungsform fallen auch unter die allgemeine Formel (AFE-II)

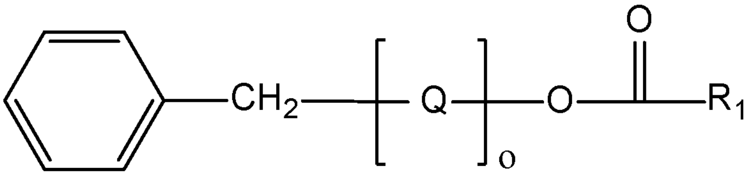

(AFE-II)

wobei

- R1 für eine gesättigte oder ungesättige $C_{11}$-$C_{29}$-Alkylgruppe steht
- o für eine ganze Zahl von 1 bis 30, bevorzugt von 1 bis 20, weiter bevorzugt von 1 bis 10 und ganz besonders bevorzugt von 1 bis 5 steht, und
- Q für eine Struktureinheit -O-CH($CH_3$)-$CH_2$- oder -O-$CH_2$-CH($CH_3$)- steht.

Im Rahmen einer weiteren ganz besonders bevorzugten Ausführungsform ist ein erfindungsgemäßes Mittel dadurch gekennzeichnet, dass es
(a3) mindestens einen alkoxylierten Fettsäureester der allgemeinen Formel (AFE-II) enthält

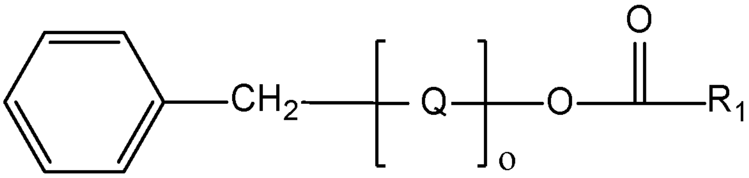

(AFE-II)

wobei

- R1 für eine gesättigte oder ungesättige $C_{11}$-$C_{29}$-Alkylgruppe steht
- o für eine ganze Zahl von 1 bis 30, bevorzugt von 1 bis 20, weiter bevorzugt von 1 bis 10 und ganz besonders bevorzugt von 1 bis 5 steht, und
- Q für eine Struktureinheit -O-CH($CH_3$)-$CH_2$- oder -O-$CH_2$-CH($CH_3$)- steht.

Auch hier kann im Fall von o größer 1 jede Struktureinheit Q unabhängig von den anderen Struktureinheiten Q gewählt werden kann.

Hierbei sind die Struktureinheiten Q sind in den alkoxylierten Fettsäureestern der allgemeinen Formel (AFE-II) so ausgerichtet, dass sich das Sauerstoffatom in der Gruppierung -O-CH($CH_3$)-$CH_2$-bwz. -O-$CH_2$-CH($CH_3$)- zur Benzylgruppe benachbart befindet, und die jeweilige Einheit - CH2- bzw. -CH(CH3)- an die Estergruppe -O-C(O)-R1 angrenzt.

Bei einer explizit ganz besonders gut geeigneten Verbindung dieses Typs handelt es sich um PPG-3 Benzyl Ether Myristate, das alterantiv auch als $\alpha$-(1-Oxotetradecyl)-$\omega$-(phenylmethoxy) Poly[oxy(methyl-1,2-ethanediyl)] bezeichnet wird und die CAS-Nummer 642443-86-5 trägt. PPG-3 Benzyl Ether Myristate kann zum Beispiel unter dem Handelsnamen Crodamol STS von der Firma Croda käuflich erworben werden.

Im Rahmen einer weiteren ganz besonders bevorzugten Ausführungsform ist ein erfindungsgemäßes Mittel dadurch gekennzeichnet, dass es
(a3) PPG-3 Benzyl Ether Myristate enthält.

Ein weiterer Typ von Anlagerungsprodukten von Ethylenoxid an die Ester aus $C_{12}$-$C_{30}$-Fettsäuren und aromatische $C_1$-$C_{12}$-Alkohole (a3) liegt dann vor, wenn ein Ester mit einem $C_1$-$C_6$-Alkylenoxid umgesetzt wird, wobei dieser Ester durch Veresterung einer $C_{12}$-$C_{30}$-Fettsäure mit mindestens einer Hydroxygruppe mit einem aromatischen $C_1$-$C_{12}$-Alkohol erhalten wurde. Beispielsweise kann Rizinusölsäure ((9Z,12R)-12-Hydroxy-9-octadecensaeure) zunächst mit Benzylalkohol, Phenol, 2-Phenylethylalkohol oder 2-Phenoxyethanol verestert werden. Bei Ausbildung dieser Addukte lagern sich nun das bzw. die $C_1$-$C_6$-Alkylenoxide an die Hydroxygruppe der Rizinusölsäure an.

Wird pro Mol Fettsäure ein Mol $C_1$-$C_6$-Alkylenoxid umgesetzt, so kann sich - wenn beispielsweise als Alkylenoxid Ethylenoxid eingesetzt wird, eine Einheit *-CH2-CH2-O-* an die Hydroyxgruppe der Rizinusölsäure addieren. Abhängig davon, in welchem molaren Überschuss das Ethylenoxid eingesetzt wird, können sich jedoch auch mehrfache Addukte ausbilden, wobei an jede Hydroxy-gruppe der Fettsäure mehrere Einheiten *-$CH_2$-$CH_2$-O-* addiert wurden, und/oder wobei auch an jede freie Hydroxygruppe des Glycerins mehrere Einheiten *-$CH_2$-$CH_2$-O-* addiert wurden. Auch diese Anlagerungsprodukt von $C_1$-$C_6$-Alkylenoxid(en) an die Ester aus $C_{12}$-$C_{30}$-Fettsäuren und aromatischen $C_1$-$C_{12}$-Alko-

holen sind erfindungsgemäß.

Die alkoxylierten Fettsäureester (a3) werden besonders bevorzugt in bestimmten Mengenbereichen im erfindungsgemäßen Mittel eingesetzt.

Besonders gute Ergebnisse wurden erhalten, wenn das Mittel - bezogen auf das Gesamtgewicht des Mittels - ein oder mehrere alkoxylierte Fettsäureester (a3) in einer Gesamtmenge 0,1 bis 20,0 Gew.-%, bevorzugt 0,5 bis 15,0 Gew.-%, weiter bevorzugt von 1,0 bis 10,0 Gew.-%, noch weiter bevorzugt von 1,0 bis 6,0 Gew.-% und ganz besonders bevorzugt von 1,0 bis 1,8 Gew.-% enthielt.

Im Rahmen einer weiteren bevorzugten Ausführungsform ist ein erfindungsgemäßes Mittel dadurch gekennzeichnet, dass es - bezogen auf das Gesamtgewicht des Mittels - ein oder mehrere alkoxylierte Fettsäureester (a3) in einer Gesamtmenge von 0,1 bis 20,0 Gew.-%, bevorzugt 0,5 bis 15,0 Gew.-%, weiter bevorzugt von 1,0 bis 10,0 Gew.-%, noch weiter bevorzugt von 1,0 bis 6,0 Gew.- % und ganz besonders bevorzugt von 1,0 bis 1,8 Gew.-% enthält.

Bei den zu dieser Erfindung führenden Arbeiten hat sich herausgestellt, dass das Vorhandensein einer oder mehrerer der zuvor beschriebenen alkoxylierten Fettsäureester (a3) die farbgebenden Verbindungen (a2), inbesondere wenn es sich hierbei um Pigmente handelt, optimal im erfindungsgemäßen Mittel dispergiert. Es wird vermutet, dass sich die auf diese Weise besonders fein im Mittel dispergierten Pigmente ganz besonders gleichmäßig und in Form eines feinen, widerstandsfähigen Films auf das Keratinmaterial auftragen lassen, was dazu führt, dass die Reibechtheiten der so erhaltenen Färbungen besonders gut sind. Weiterhin wird vermutet, dass diese besonders feine Dispergierung der Pigmente im Mittel auch für die guten Waschechtheiten verantwortlich ist, die mit den erfindungsgemäßen Mitteln erzielt werden können.

Es wurden somit sehr gute Ergebnisse erhalten, wenn das oder die alkoxylierten Fettsäureester (a3) als die wesentlichen Dispergierhilfen bzw. Additive im erfindungsgemäßen Mittel enthalten waren. Weiterführende Arbeiten haben darüberhinaus gezeigt, dass diese Wirkung noch weiter verstärkt werden kann, wenn den Mitteln noch ein weiters $C_1$-$C_6$-Alkylenoxid-Additionsprodukt (a4) hinzugesetzt wird.

<u>Anlagerungsprodukte von $C_1$-$C_6$-Alkylenoxid(en) an aliphatische $C_1$-$C_{24}$-Alkanole (a4)</u>

Bei der als weiterem Dispergieradditiv besonders wirksamen Substanzgruppe (a4) handelt es sich um die Anlagerungsprodukte von $C_1$-$C_6$-Alkylenoxiden an C1-C24-Alkanole.

Im Folgenden werden die Anlagerungsprodukte von $C_1$-$C_6$-Alkylenoxid(en) an aliphatische $C_1$-$C_{24}$-Alkanole (a4) verkürzt auch als alkoxylierte Alkanole bezeichnet.

Die Anlagerungsprodukte von $C_1$-$C_6$-Alkylenoxid(en) an aliphatische $C_1$-$C_{24}$-Alkanole (a4) sind die Verbindungen der allgemeinen Formel (AA-I)

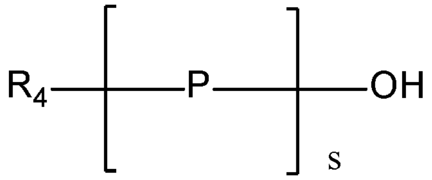

(AA-I)

worin

R4 für eine gesättigte oder ungesättigte $C_1$-$C_{12}$-Alkylgruppe steht und
P für eine Struktureinheit $-O-CH_2-CH_2-$ , $-O-CH(CH_3)-CH_2-$ oder $-O-CH_2-CH(CH_3)-$steht, und
s für eine ganze Zahl von 1 bis 60 steht.

Der Rest R4 steht für eine gesättigte oder ungesättigte, $C_1$-$C_{12}$-Alkylgruppe Ungesättigt können die Reste R4 ab einer Kohlenstoffanzahl von mindestens 2 C-Atomen sein. Eine ungesättigte Alkylgruppe kann eine oder mehrere Doppelbindungen umfassen und wird alternativ auch als $C_2$-$C_{12}$-Alkenylgruppe bezeichnet. Die gesättigte oder ungesättige $C_1$-$C_{12}$-Alkylgruppe kann linear oder verzweigt sein.

Besonders bevorzugt steht der Rest R4 für eine gesättigte, unverzweigte $C_1$-$C_{12}$-Alkylgruppe. Ganz besonders bevorzgt steht der Rest R4 für eine gesättigte, unverzweigte $C_1$-$C_6$-Alkylgruppe.

In den Verbindungen der Formel (AA-I) steht P für eine Struktureinheit $-O-CH_2-CH_2-$ , $-O-CH(CH_3)-CH_2-$ oder $-O-CH_2-CH(CH_3)-$ steht. Die Anzahl der in den Verbindungen der Formel (AA-I) enthaltenen Struktureinheiten ergibt sich durchdie Indexzahl s. Hierbei sind die Struktureinheiten P so ausgerichtet, dass sich das Sauerstoffatom in jeder Gruppierung $-O-CH_2-CH_2-$, $-O-CH(CH_3)-CH_2-$ und $-O-CH_2-CH(CH_3)-$ zum Alkylrest R4 benachbart befindet, und die jeweilige Einheit -CH2- bzw. - CH(CH3)- an die Hyroxy-Gruppe -OH angrenzt.

Wenn s für eine Zahl von größer als 1 steht, sind in den Verbindungen der Formel (AA-I) mehrere Struktureinheiten P enthalten. In diesem Fall kann jede Struktureinheit P von den anderen Struktureinheiten P unabhängig gewählt werden.

Die Indexzahl s steht für eine ganze Zahl von 1 bis 60, bevorzugt für eine ganze Zahl von 1 bis 40, weiter bevorzugt für eine ganze Zahl von 10 bis 30 und ganz besonders bevorzugt für eine ganze Zahl von 10 bis 20.

Daher ist ein erfindungsgemäßes Mitte weiterhin dadurch gekennzeichnet dass es

(a4) mindestens ein Anlagerungsprodukt von $C_1$-$C_6$-Alkylenoxid(en) an aliphatische C1-C24-Alkanole der Formel (AA-I) enthält,

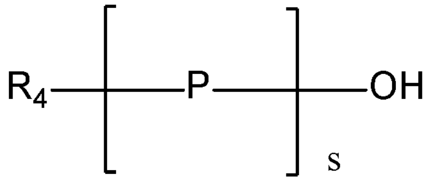

(AA-I)

worin

- R4 für eine gesättigte oder ungesättigte $C_1$-$C_{12}$-Alkylgruppe, besonders bevorzugt für eine $C_1$-$C_6$-Alkylgruppe, steht und
- P für eine Struktureinheit $-O-CH_2-CH_2-$ , $-O-CH(CH_3)-CH_2-$ oder $-O-CH_2-CH(CH_3)$-steht, und
- s für eine ganze Zahl von 1 bis 60, bevorzugt für eine ganze Zahl von 1 bis 40, weiter bevorzugt für eine ganze Zahl von 10 bis 30 und ganz besonders bevorzugt für eine ganze Zahl von 10 bis 20, steht.

Bei einem ganz besonders gut geeigneten Anlagerungsprodukt von $C_1$-$C_6$-Alkylenoxid(en) an aliphatische $C_1$-$C_{24}$-Alkanole der Formel (AA-I) handelt es sich um Propylene Glycol Monobutylether, das auch als PPG-14 Butyl ether bezeichnet wird und die CAS-Nummer 9003-13-8. Trägt. PPG-14 Butyl ether kann kommerziell unter dem Handelsnamen Ucon Fluid AP von der Firma Dow erworben werden.

Im Rahmen einer weiteren ganz besonders bevorzugten Aufführungsform ist ein erfindungsgemäßes Mittel daher weiterhin dadurch gekennzeichnet dass es

(a4) PPG-14 Butyl ether enthält.

Die alkoxylierten Alkanole (a4) werden besonders bevorzugt in bestimmten Mengenbereichen im erfindungsgemäßen Mittel eingesetzt.

Besonders gute Ergebnisse wurden erhalten, wenn das Mittel - bezogen auf das Gesamtgewicht des Mittels - ein oder mehrere alkoxylierten Alkanole (a4) in einer Gesamtmenge von 0,1 bis 20,0 Gew.-%, bevorzugt 0,2 bis 15,0 Gew.-%, weiter bevorzugt von 0,3 bis 10,0 Gew.-%, noch weiter bevorzugt von 0,4 bis 5,0 Gew.-% nd ganz besonders bevorzugt von 0,5 bis 3,0 Gew.-% enthielt.

Im Rahmen einer weiteren bevorzugten Ausführungsform ist ein erfindungsgemäßes Mittel dadurch gekennzeichnet, dass es - bezogen auf das Gesamtgewicht des Mittels - ein oder mehrere Anlagerungsprodukte von $C_1$-$C_6$-Alkylenoxid(en) an aliphatische $C_1$-$C_{24}$-Alkanole (a4) in einer Gesamtmenge von 0,1 bis 20,0 Gew.-%, bevorzugt 0,2 bis 15,0 Gew.-%, weiter bevorzugt von 0,3 bis 10,0 Gew.-%, noch weiter bevorzugt von 0,4 bis 5,0 Gew.-%, noch weite bevorzugt von 0,5 bis 3,0 Gew.-% und ganz besonders bevorzugt von 0,5 bis 1,5 Gew.-% enthält.

<u>pH-Wert der Mittel</u>

Die pH-Werte des erfindungsgemäßen Mittels wird bevorzugt auf einen leicht sauren bis alkalischen pH-Wert eingestellt. Ganz besonders bevorzugt besitzt das Mittel einen alkalischen pH-Wert im Bereich von 3,8 bis 11,5, bevorzugt von 4,0 bis 10,0, und weiter bevorzugt von 5,0 bis 9,0 und ganz besonders bevorzugt von 6,0 bis 8,0

Im Rahmen einer weiteren besonders bevorzugten Ausführungsform ist ein erfindungsgemäßes Mittel dadurch gekennzeichnet, dass es Wasser enthält und einen pH-Wert von 3,8 bis 11,5 bevorzugt von 4,0 bis 10,0, weiter bevorzugt von 5,0 bis 9,0 und ganz besonders bevorzugt von 6,0 bis 8,0 besitzt.

Zur Einstellung des gewünschten pH-Wertes kann das erfindungsgemäße Mittel mindestens ein Acidifizierungsmittel und/oder Alkalisierungsmittel enthalten. Bei den pH-Werten im Sinne der vorliegenden Erfindung handelt es sich um pH-Werte, die bei einer Temperatur von 22°C gemessen wurden.

Als Alkalisierungsmittel können die Mittel beispielsweise Ammoniak, Alkanolamine und/oder basische Aminosäuren enthalten.

Die in dem erfindungsgemäßen Mittel einsetzbaren Alkanolamine werden bevorzugt ausgewählt aus primären

Aminen mit einem $C_2$-$C_6$-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol.

Erfindungsgemäß besonders bevorzugte Alkanolamine werden ausgewählt aus 2-Aminoethan-1-ol und/oder 2-Amino-2-methylpropan-1-ol. Eine besonders bevorzugte Ausführungsform ist daher dadurch gekennzeichnet, dass das erfindungsgemäße Mittel als Alkalisierungsmittel ein Alkanolamin ausgewählt aus 2-Aminoethan-1-ol und/oder 2-Amino-2-methylpropan-1-ol enthält.

Als Aminosäure im Sinne der Erfindung gilt eine organische Verbindung, die in ihrer Struktur mindestens eine protonierbare Aminogruppe und mindestens eine -COOH- oder eine -$SO_3H$-Gruppe enthält. Bevorzugte Aminosäuren sind Aminocarbonsäuren, insbesondere $\alpha$-(alpha)-Aminocarbonsäuren und $\omega$-Aminocarbonsäuren, wobei $\alpha$-Aminocarbonsäuren besonders bevorzugt sind.

Unter basischen Aminosäuren sind erfindungsgemäß solche Aminosäuren zu verstehen, welche einen isoelektrischen Punkt pl von größer 7,0 besitzen.

Basische $\alpha$-Aminocarbonsäuren enthalten mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist ein erfindungsgemäßes Mittel daher dadurch gekennzeichnet, dass es sich bei dem Alkalisierungsmittel um eine basische Aminosäure aus der Gruppe Arginin, Lysin, Ornithin und/oder Histidin handelt.

Darüber hinaus kann das Mittel weitere Alkalisierungsmittel, insbesondere anorganische Alkalisierungsmittel enthalten. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Ganz besonders bevorzugte Alkalisierungsmittel sind Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

In einer weiteren ganz besonders bevorzugten Ausführungsform ist ein erfindungsgemäßes Verfahren dadurch gekennzeichnet, dass das Färbemittel mindestens ein Alkalisierungsmittel aus der Gruppe aus Ammoniak, 2-Aminoethan-1-ol (Monoethanolamin), 3-Aminopropan-1-ol, 4-Amino-butan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat enthält.

Zur Einstellung des gewünschten pH-Wertes kann das erfindungsgemäße Mittel auch mindestens ein ein Puffersystem aus mindestens einer anorganischen oder organischen Säure und mindestens einem Salz dieser Säure enthalten.

Eine ganz besonders gut geeignete anorganische Säure ist hierbe Kaliumdihydrogenphosphat Kaliumdihydrogenphosphat besitzt die Summenformel $KH_2PO_4$ und trägt die CAS-Nummer 7778-77-0. Kaliumdihydrogenphosphat besitzt eine molare Masse von 136,09 g/mol. Es ist gut löslich in Wasser (222 g/l bei 20 °C) und reagiert in Wasser sauer. Eine 5 %ige Lösung von Kaliumdihydrogenphosphat in Wasser besitzt einen pH-Wert von 4,4.

Eine weitere ganz besonders gut geeignete anorganische Säure (b2-I) ist Natriumdihydrogenphosphat. Natriumdihydrogenphosphat besitzt die Summenformel $NaH_2PO_4$ und trägt die CAS-Nummern 7558-80-7 (Anhydrat), 10049-21-5 (Monohdrat) und 13472-35-0 (Dihydrat). Das wasserfreie Natriumdihydrogenphosphat besitzt eine molare Masse von 119,98 g/mol. Natriumdihydrogenphosphat reagiert in wässriger Lösung sauer.

Besonders bevorzugt als korrespondierendes Salz der vorgenannten beiden Säuren ist Dikaliumhydrogenphosphat. Dikaliumhydrogenphosphat besitzt die Summenformel $K_2HPO_4$ und trägt die CAS-Nummern 7758-11-4 (wasserfrei) und 16788-57-1 (Trihydrat). Das wasserfreie Dikaliumhydrogenphosphat besitzt eine molare Masse von 174,18 g/mol. Dikaliumhydrogenphosphat reagiert in wässriger Lösung alkalisch.

Ebenfalls besonders bevorzugt als korresprondierdens Salz der vorgenannten beiden Säuren (b2-II) ist Dinatriumhydrogenphosphat. Dinatriumhydrogenphosphat besitzt die Summenformel $Na_2HPO_4$ und trägt die CAS-Nummern 7558-79-4 (wasserfrei), 10028-24-7 (Dihydrat), 7782-85-6 (Heptahydrat) und 10039-32-4 (Dodecahydrat).

Das wasserfreie Dinatriumhydrogenphosphat besitzt eine molare Masse von 141,96 g/mol. Dinatriumhydrogenphosphat reagiert in wässriger Lösung alkalisch.

weitere optionale Rezepturbestandteile im Mittel

Zusätzlich zu den bereits beschriebenen erfindungswesentlichen bzw. besonders bevorzugten Bestandteilen (a1), (a2), (a3) sowie gegebenenfalls (a4) kann das Mittel zusätzlich auch noch weitere optionale Inhaltsstoffe enthalten.

Als weiteren optionalen Bestandteil kann das erfindungsgemäße Mittel zusätzlich auch noch mindestens einen Fettbestandteil enthalten. Geeignete Fettbestandteile können ausgewählt werden aus der Gruppe der $C_{12}$-$C_{24}$-Fettalkohole, der $C_{12}$-$C_{24}$-Fettsäuretriglyceride, der $C_{12}$-$C_{24}$-Fettsäure-monoglyceride, der $C_{12}$-$C_{24}$-Fettsäurediglyceride und/oder der Kohlenwasserstoffe.

Bei den $C_{12}$-$C_{24}$-Fettalkoholen kann es sich um gesättigte, ein- oder mehrfach ungesättigte, lineare oder verzweigte Fettalkohole mit 12 bis 24 C-Atomen handeln.

Beispiele für bevorzugte lineare, gesättigte C12-$C_{24}$-Fettalkohole sind Dodecan-1-ol (Dodecylalkohol, Laurylalkohol), Tetradecan-1-ol (Tetradecylalkohol, Myristylalkohol), Hexadecan-1-ol (Hexadecylalkohol, Cetylalkohol, Palmitylalkohol), Octadecan-1-ol (Octadecylalkohol, Stearylalkohol), Arachylalkohol (Eicosan-1-ol), Heneicosylalkohol (Heneicosan-1-ol) und/oder Behenylalkohol (Docosan-1-ol).

Bevorzugte lineare, ungesättigte Fettalkohole sind (9Z)-Octadec-9-en-1-ol (Oleylalkohol), (9E)-Octadec-9-en-1-ol (Elaidylalkohol), (9Z,12Z)-Octadeca-9,12-dien-1-ol (Linoleylalkohol), (92,12Z,15Z)-Octadeca-9,12,15-trien-1-ol (Linolenoylalkohol), Gadoleylalkohol ((9Z)-Eicos-9-en-1-ol), Arachidonalkohol ((5*Z*,8*Z*,11*Z*,14*Z*)-Eicosa-5,8,11,14-tetraen-1-ol), Erucylalkohol ((13Z)-Docos-13-en-1-ol) und/oder Brassidylalkohol ((13*E*)-Docosen-1-ol).

Die bevorzugten Vertreter für verzweigte Fettalkohole sind 2-Octyl-dodecanol, 2-Hexyl-Dodecanol und/oder 2-Butyl-dodecanol. Es hat sich als ganz besonders bevorzugt erwiesen, ein oder mehrere $C_{12}$-$C_{24}$-Fettalkohole in ganz bestimmten Mengenbereichen einzusetzen.

Es ist ganz besonders bevorzugt, wenn die das Mittel - bezogen auf das Gesamtgewicht des Mittels - einen oder mehrere $C_{12}$-$C_{24}$-Fettalkohole in einer Gesamtmenge von 2,0 bis 50,0 Gew.-%, bevorzugt von 3,0 bis 30,0 Gew.-%, weiter bevorzugt von 4,0 bis 20,0 Gew.-%, noch weiter bevorzugt von 5,0 bis 15,0 Gew.-% und ganz besonders bevorzugt von 5,0 bis 10,0 Gew.-% enthält.

Weiterhin kann das Mittel auch mindestes ein Konservierungsmittel enthalten. Als Konservierungsmittel können die folgenden Substanzen und deren Mischungen Verwendung finden:

- aromatische Alkohole, wie beispielsweise Phenoxyethanol, Benzylalkohol, Phenethylalkohol, Phenoxyisopropanol,
- Aldehyde wie beispielsweise Formaldehydlösung und Paraformaldehyd, Glutaraldehyd
- Parabene, beispielsweise Methylparaben, Ethylparaben, Propylparaben, Butylparaben, Isobutylparaben
- 1,2-Alkandiole mit 5 bis 22 Kohlenstoffatomen in der Kohlenstoffkette, wie beispielsweise 1,2-Pentandiol, 1,2-Hexandiol, 1,2-Heptandiol, 1,2-Dekandiol, 1,2-Dodekandiol, 1,2-Hexadekandiol,
- Formaldehyd abspaltende Verbindungen, wie beispielsweise DMDM Hydantoin, Diazolidinyl Urea
- Halogenierte Verbindungen wie beispielsweise Isothiazolinone, wie beispielsweise Methylchloroisothiazolinon / Methylisothiazolinone, Triclosan, Triclocarban, lodopropynylbutylcarbamat, 5-Bromo-5-Nitro-1,3-Dioxan, Chlorhexidindigluconat und Chlorhexidinacetat, 2-Bromo-2-Nitropropan-1,3-diol, Methyldibromoglutaronitril,
- Anorganische Verbindungen wie beispielsweise Sulfite, Borsäure und Borate, Bisulfite,
- Kationische Substanzen wie beispielsweise Quaternium-15, Benzalkoniumchlorid, Benzethoniumchlorid, Polyaminopropylbiguanid,
- Organische Säuren und deren physiologisch verträgliche Salze wie beispielsweise Citronensäure, Milchsäure, Essigsäure, Benzoesäure, Sorbinsäure, Salicylsäure, Dehydroacetsäure
- Aktive Wirkstoffe mit zusätzlichen Wirkungen wie beispielsweise Zink-Pyrithion, Piroctonolamin,
- Antioxidantien wie beispielsweise BHT (butyliertes Hydroxytoluol), BHA (butyliertes Hydroxyanisol), Propylgallat, t-Butylhydrochinon,
- Komplexbildner wie beispielsweise EDTA und dessen Derivate, HEDTA und dessen Derivate, Etidronic Acid und deren Salze.

Bei Bedarf können die erfindungsgemäßen Mittel zusätzlich auch mindestens eine oberflächenaktive Substanz enthalten, wobei solche oberflächenaktive Substanzen je nach Anwendungsgebiet als Tenside oder als Emulgatoren bezeichnet werden: Sie sind bevorzugt aus anionischen, kationischen, zwitterionischen, amphoteren und nichtionischen Tensiden und Emulgatoren ausgewählt.

Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Die anionischen Tenside werden in Anteilen von 0,1 bis 45 Gew.%, bevorzugt 1 bis 30 Gew.% und ganz besonders bevorzugt von 1 bis 15 Gew.%, bezogen

auf die Gesamtmenge des anwendungsbereiten Mittels, eingesetzt.

Bevorzugte zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethylimidazoline. Ein bevorzugtes zwitterionisches Tensid ist unter der INCI-Bezeichnung Cocamidopropyl Betaine bekannt.

Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und $C_{12}$-$C_{18}$-Acylsarcosin.

Bevorzugte nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Bevorzugte quaternäre Ammoniumverbindungen sind Ammoniumhalogenide sowie die unter den INCI-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Eine erfindungsgemäß besonders geeignete Verbindung aus Gruppe der Amidoamine stellt das unter der Bezeichnung Tegoamid® S 18 im Handel erhältliche Stearamidopropyl-dimethylamin dar. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Die kationischen Tenside sind in den erfindungsgemäß verwendeten Mitteln bevorzugt in Anteilen von 0,05 bis 10 Gew.-%, bezogen auf das gesamte Mittel, enthalten.

Die Tenside werden bevorzugt in Anteilen von 0,1 bis 45 Gew.%, bevorzugt 1 bis 30 Gew.% und ganz besonders bevorzugt von 1 bis 15 Gew.%, bezogen auf die Gesamtmenge der Mittel, eingesetzt.

Weiterhin können die erfindungsgemäßen Mitte zusätzlich auch noch mindestens ein Lösungsmittel enthalten. Besonders gut geeignete Lösungsmittel können ausgewählt werden aus der Gruppe aus 1,2-Propandiol, 1,3-Propandiol, Ethanol, Isopropanol, Diethylenglycolmonoethylether, Glycerin, 1,2-Butylenglycol, Dipropylenglycol, Ethylencarbonat, Propylencarbonat, 2-Phenoxyethanol und Benzylalkohol.

Die Lösungmittel werden bevorzugt in Anteilen von 0,1 bis 45 Gew.%, bevorzugt 1 bis 30 Gew.% und ganz besonders bevorzugt von 1 bis 15 Gew.%, bezogen auf die Gesamtmenge der Mittel, eingesetzt.

Zur Einstellung der gewünschten Viskosität bzw. des optimalen Fließverhaltens können die erfindungsgemäßen Mittel als weiteren optionalen Bestandteil zusätzlich auch noch mindestens einen polymeren Verdicker enthalten.

Im folgenden werden einige Beispiele typischer polymerer Verdicker für wäßrige bzw. wasserhaltige Systeme aufgeführt:

Acrylamides Copolymer, Acrylamide/Sodium Acrylate Copolymer, Acrylamide/Sodium Acryloyldimethyltaurate Copolymer, Acrylates/Acetoacetoxyethyl Methacrylate Copolymer, Acrylates/Beheneth-25 Methacrylate Copolymer, Acrylates/$C_{10}$-$_{30}$ Alkyl Acrylate Crosspolymer, Acrylates/Ceteth-20 Itaconate Copolymer, Acrylates/Ceteth-20 Methacrylate Copolymer, Acrylates/Laureth-25 Methacrylate Copolymer, Acrylates/Palmeth-25 Acrylate Copolymer, Acrylates/Palmeth-25 Itaconate Copolymer, Acrylates/Steareth-50 Acrylate Copolymer, Acrylates/Steareth-20 Itaconate Copolymer, Acrylates/Steareth-20 Methacrylate Copolymer, Acrylates/Stearyl Methacrylate Copolymer, Acrylates/Vinyl Isodecanoate Crosspolymer, Acrylic Acid/Acrylonitrogens Copolymer, Agar, Agarose, Alcaligenes Polysaccharides, Algin, Alginic Acid, Ammonium Acrylates/Acrylonitrogens Copolymer, Ammonium Acrylates Copolymer, Ammonium Acryloyldimethyltaurate/Vinyl Formamide Copolymer, Ammonium Acryloyldimethyltaurate/VP Copolymer, Ammonium Alginate, Ammonium Polyacryloyldimethyl Taurate, Amylopectin, Ascorbyl Methylsilanol Pectinate, Astragalus Gummifer Gum, Attapulgite, Avena Sativa (Oat) Kernel Flour, Bentonite, Butoxy Chitosan, Caesalpinia Spinosa Gum, Calcium Alginate, Calcium Carboxymethyl Cellulose, Calcium Carrageenan, Calcium Potassium Carbomer, Calcium Starch Octenylsuccinate, C20-40 Alkyl Stearate, Carbomer, Carboxybutyl Chitosan, Carboxymethyl Chitin, Carboxymethyl Chitosan, Carboxymethyl Dextran, Carboxymethyl Hydroxyethylcellulose, Carboxymethyl Hydroxypropyl Guar, Cellulose Acetate Propionate Carboxylate, Cellulose Gum, Ceratonia Siliqua Gum, Cetyl Hydroxyethylcellulose, Cholesterol/HDI/Pullulan Copolymer, Cholesteryl Hexyl Dicarbamate Pullulan, Cyamopsis Tetragonoloba (Guar) Gum, Diglycol/CHDM/Isophthalates/SIP Copolymer, Dihydrogenated Tallow Benzylmonium Hectorite, Dimethicone Crosspolymer-2, Dimethicone Propyl PG-Betaine, DMAPA Acrylates/Acrylic Acid/Acrylonitrogens Copolymer, Ethylene/Sodium Acrylate Copolymer, Gelatin, Gellan Gum, Glyceryl Alginate, Glycine Soja (Soybean) Flour, Guar Hydroxypropyltrimonium Chloride, Hectorite, Hydrated Silica, Hydrogenated Potato Starch, Hydroxybutyl Methylcellulose, Hydroxyethyl Acrylate/Sodium Acryloyldimethyl Taurate Copolymer, Hydroxyethylcellulose, Hydroxyethyl Chitosan, Hydroxyethyl Ethylcellulose, Hydroxypropylcellulose, Hydroxypropyl Chitosan, Hydroxypropyl Ethylenediamine Carbomer, Hydroxypropyl Guar, Hydroxypropyl Methylcellulose, Hydroxypropyl Methylcellulose Stearoxy Ether, Hydroxypropyl Starch, Hydroxypropyl Starch Phosphate, Hydroxypropyl Xanthan Gum, Hydroxystearamide MEA, Isobutylene/Sodium Maleate Copolymer, Lithium Magnesium Silicate, Lithium Magnesium Sodium Silicate, Macrocystis Pyrifera (Kelp), Magnesium Alginate, Magnesium Aluminum Silicate, Magnesium Silicate, Magnesium Trisilicate, Methoxy PEG-22/Dodecyl Glycol Copolymer, Methylcellulose,

Methyl Ethylcellulose, Methyl Hydroxyethylcellulose, Microcrystalline Cellulose, Montmorillonite, Moroccan Lava Clay, Natto Gum, Nonoxynyl Hydroxyethylcellulose, Octadecene/MA Copolymer, Pectin, PEG-800, PEG-Crosspolymer, PEG-150/Decyl Alcohol/SMDI Copolymer, PEG-175 Diisostearate, PEG-190 Distearate, PEG-15 Glyceryl Tristearate, PEG-140 Glyceryl Tristearate, PEG-240/HDI Copolymer Bis-Decyltetradeceth-20 Ether, PEG-100/IPDI Copolymer, PEG-180/Laureth-50/TMMG Copolymer, PEG-10/Lauryl Dimethicone Crosspolymer, PEG-15/Lauryl Dimethicone Crosspolymer, PEG-2M, PEG-5M, PEG-7M, PEG-9M, PEG-14M, PEG-20M, PEG-23M, PEG-25M, PEG-45M, PEG-65M, PEG-90M, PEG-115M, PEG-160M, PEG-120 Methyl Glucose Trioleate, PEG-180/Octoxynol-40/TMMG Copolymer, PEG-150 Pentaerythrityl Tetrastearate, PEG-4 Rapeseedamide, PEG-150/Stearyl Alcohol/SMDI Copolymer, Polyacrylate-3, Polyacrylic Acid, Polycyclopentadiene, Polyether-1, Polyethylene/Isopropyl Maleate/MA Copolyol, Polymethacrylic Acid, Polyquaternium-52, Polyvinyl Alcohol, Potassium Alginate, Potassium Aluminum Polyacrylate, Potassium Carbomer, Potassium Carrageenan, Potassium Polyacrylate, Potato Starch Modified, PPG-14 Laureth-60 Hexyl Dicarbamate, PPG-14 Laureth-60 Isophoryl Dicarbamate, PPG-14 Palmeth-60 Hexyl Dicarbamate, Propylene Glycol Alginate, PVP/Decene Copolymer, PVP Montmorillonite, Rhizobian Gum, Ricinoleic Acid/Adipic Acid/AEEA Copolymer, Sclerotium Gum, Sodium Acrylate/Acryloyldimethyl Taurate Copolymer, Sodium Acrylates/Acrolein Copolymer, Sodium Acrylates/Acrylonitrogens Copolymer, Sodium Acrylates Copolymer, Sodium Acrylates/Vinyl Isodecanoate Crosspolymer, Sodium Acrylate/Vinyl Alcohol Copolymer, Sodium Carbomer, Sodium Carboxymethyl Chitin, Sodium Carboxymethyl Dextran, Sodium Carboxymethyl Beta-Glucan, Sodium Carboxymethyl Starch, Sodium Carrageenan, Sodium Cellulose Sulfate, Sodium Cyclodextrin Sulfate, Sodium Hydroxypropyl Starch Phosphate, Sodium Isooctylene/MA Copolymer, Sodium Magnesium Fluorosilicate, Sodium Polyacrylate, Sodium Polyacrylate Starch, Sodium Polyacryloyldimethyl Taurate, Sodium Polymethacrylate, Sodium Polystyrene Sulfonate, Sodium Silicoaluminate, Sodium Starch Octenylsuccinate, Sodium Stearoxy PG-Hydroxyethylcellulose Sulfonate, Sodium Styrene/Acrylates Copolymer, Sodium Tauride Acrylates/Acrylic Acid/Acrylonitrogens Copolymer, Solanum Tuberosum (Potato) Starch, Starch/Acrylates/Acrylamide Copolymer, Starch Hydroxypropyltrimonium Chloride, Steareth-60 Cetyl Ether, Steareth-100/PEG-136/HDI Copolymer, Sterculia Urens Gum, Synthetic Fluorphlogopite, Tamarindus Indica Seed Gum, Tapioca Starch, TEA-Alginate, TEA-Carbomer, Triticum Vulgare (Wheat) Starch, Tromethamine Acrylates/Acrylonitrogens Copolymer, Tromethamine Magnesium Aluminum Silicate, Welan Gum, Xanthan Gum, Yeast Beta-Glucan, Yeast Polysaccharides, Zea Mays (Corn) Starch.

Der bzw. die polymeren Verdicker werden bevorzugt in Anteilen von 0,1 bis 45 Gew.%, bevorzugt 1 bis 30 Gew.% und ganz besonders bevorzugt von 1 bis 15 Gew.%, bezogen auf die Gesamtmenge der Mittel, eingesetzt.

Ferner können die erfindungsgemäßen Mittel weitere Wirk-, Hilfs- und Zusatzstoffe enthalten, beispielsweise nichtionische Polymere (wie Vinylpyrrolidinon/Vinylacrylat-Copolymere, Polyvinylpyrrolidinon und Vinylpyrrolidinon/Vinylacetat-Copolymere, Polyethylenglykole und Polysiloxane); kationische Polymere (wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol); zwitterionische und amphotere Polymere (wie Acrylamidopropyl-trimethylammoniumchlorid/Acrylat-Copolymere und Octylacrylamid/Methylmethacrylat/t-Butylaminoethylmethacrylat/2-Hydroxypropylmethacrylat-Copolymere); anionische Polymere (wie Polyacrylsäuren, vernetzte Polyacrylsäuren, Vinylacetat/Crotonsäure-Copolymere, Vinylpyrrolidinon/Vinylacrylat-Copolymere, Vinylacetat/ Butylmaleat/Isobornylacrylat-Copolymere, Methylvinylether/Malein-säureanhydrid-Copolymere und Acrylsäure/Ethylacrylat/N-t-Butyl-acrylamid-Terpolymere); weitere Verdickungsmittel (wie Agar-Agar, Guar-Gum, Alginate, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol); Strukturanten (wie Glucose, Maleinsäure und Milchsäure); haarkonditionierende Verbindungen (wie Phospholipide, Sojalecithin, Ei-Lecitin und Kephaline sowie Silikonöle); Proteinhydrolysate (insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate); faserstrukturverbessernde Wirkstoffe (insbesondere Mono-, Di- und Oligosaccharide wie beispielsweise Glucose, Galactose, Fructose, Fruchtzucker und Lactose); Entschäumer (wie Silikone, bevorzugt Dimethicon); Farbstoffe zum Anfärben des Mittels; Antischuppenwirkstoffe (wie Piroctone Olamine, Zink Omadine und Climbazol); Lichtschutzmittel beziehungsweise UV-Blocker (insbesondere derivatisierte Benzophenone, Zimtsäure-Derivate und Triazine); Wirkstoffe (wie Panthenol, Pantothensäure, Pantolacton, Allantoin, Pyrrolidinoncarbonsäuren und deren Salze sowie Bisabolol); Vitamine, Provitamine und Vitaminvorstufen (insbesondere solche der Gruppen A, $B_3$, $B_5$, $B_6$, C, E, F und H); Cholesterin; Konsistenzgeber (wie Zuckerester, Polyolester oder Polyolalkylether); Fettsäurealkanolamide; Quell- und Penetrationsstoffe (wie Glycerin, Propylenglykolmonoethylether, Carbonate, Hydrogencarbonate, Guanidine, Harnstoffe sowie primäre, sekundäre und tertiäre Phosphate); Trübungsmittel (wie Latex, Styrol/PVP- und Styrol/Acrylamid-Copolymere); Perlglanzmittel (wie Ethylenglykolmono- und - distearat sowie PEG-3-distearat); Pigmente; Parfümöle; Treibmittel (wie Propan-Butan-Gemische, $N_2O$, Dimethylether, $CO_2$ und Luft) sowie Antioxidantien.

Die Auswahl dieser weiteren Stoffe wird der Fachmann gemäß der gewünschten Eigenschaften der Mittel

treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Wassergehalt im Mittel

Bei dem zuvor beschriebenen Mittel handelt es sich um ein anwendungsbereites Mittel, welches auf das keratinische Material appliziert werden kann. Dieses anwendungsbereite Mittel besitzt bevorzugt einen hohen Wasseranteil. Es hat sich herausgestellt, dass besonders die Mittel besonders gut geeignet sind, die - bezogen auf das Gesamtgewicht des Mittels - 50,0 bis 98,0 Gew.-%, bevorzugt 60,0 bis 90,0 Gew.-% und besonders bevorzugt 70,0 bis 90,0 Gew.-% Wasser enthalten.
In einer weiteren explizit ganz besonders bevorzugten Ausführungsform ist ein erfindungsgemäßes Mittel dadurch gekennzeichnet, dass es - bezogen auf das Gesamtgewicht des Mittels - 50,0 bis 98,0 Gew.-%, bevorzugt 60,0 bis 90,0 Gew.-% und besonders bevorzugt 70,0 bis 90,0 Gew.-% Wasser enthält.

Verfahren zum Färben von Keratinmaterial

Die zuvor beschriebenen Mittel lassen sich hervorragend in Verfahren zum Färben von keratinischem Material, insbesondere von menschlichen Haaren einsetzen.
Ein zweiter Gegenstand der vorliegenden Erfindung ist daher ein Verfahren zum Färben von keratinischem Material, insbesondere menschlichen Haaren, umfassend die folgenden Schritte:

(1) Anwendung eines Färbemittels auf dem keratinischem Material, wobei das Färbemittel ein Mittel ist, wie es bei der Beschreibung der ersten Erfindungsgegenstand im Detail offenbart wurde,
(2) Einwirken des Färbemittels auf dem keratinischen Material und
(3) Ausspülen des Färbemittels.

In Schritt (1) des erfindungsgemäßen Verfahrens wird das Mittel des ersten Erfindungsgegenstand auf dem keratinische Material, bei dem es sich ganz besonders bevorzugt um menschliche Haare handelt, angewendet.
In Schritt (2) des erfindungsgemäßen Verfahrens wird das Mittel dann nach seiner Applikation auf das keratinische Material einwirken gelassen. In diesem Zusammenhang sind verschiedene Einwirkzeiten von beispielsweise 30 Sekunden bis 60 Minuten denkbar.
Ein großer Vorteil des erfindungsgemäßen Färbesystems liegt jedoch darin, dass auch in sehr kurzen Zeiträumen nach kurzen Einwirkzeiten ein intensive Farbergebnis erzielt werden kann. Aus diesem Grund ist es von Vorteil, wenn die Anwendungsmischung nach ihrer Applikation nur für vergleichsweise kurze Zeiträume von 30 Sekunden bis 15 Minuten, bevorzugt von 30 Sekunden bis 10 Minuten, und besonders bevorzugt von 1 bis 5 Minuten auf dem Keratinmaterial verbleibt.
In einer weiteren bevorzugten Ausführungsform ist ein erfindungsgemäßes Verfahren gekennzeichnet durch das
(2) Einwirken des Färbemittels auf dem keratinischen Material für einen Zeitraum von 30 Sekunden bis 15 Minuten, bevorzugt von 30 Sekunden bis 10 Minuten, und besonders bevorzugt von 1 bis 5 Minuten.
Im Anschluss an das Einwirken der Anwendungsmischung auf das Keratinmaterial wird diese schließlich in Schritt (3) des Verfahrens ausgespült.
Hierbei kann die Awendungsmischung in einer Ausführungsform nur mit Wasser, d.h. ohne Zuhilfenahme eines Nachbehandlungsmittels oder eines Shampoos, ausgewaschen werden. Auch die Anwendung eines Nachbehandlungsmittels oder Conditioners nach Schritt (3) ist prinzipiell denkbar.

Verfahren zum Färben von Keratinmaterial, bei welchem zunächst das anwendungsbereite Mittel hergestellt wird.

Wie bereits zuvor beschrieben, handelt es sich bei dem Mittel des ersten Erfindungsgegenstands um ein anwendungsbereits Mittel, das dem Anwender entweder direkt in seiner anwendungsbereiten Form zur Verfügung gestellt wird, oder aber das erst kurz vor der Anwendung durch Vermischen von verschiedenen Mitteln hergestellt wird.
Um eine besonders feine Verteilung der Pigmente zu gewährleisten, hat es sich als ganz besonders bevorzugt herausgestellt, das anwendungsbereite Mittel kurz vor der Anwendung durch Vermischen von zwei oder drei verschiedenen Mitteln herzustellen.
Im Rahmen einer besonders bevorzugten Ausführungsform wird das anwendungsbereite Mittel demnach durch Vermischen von mindestens zwei verschiedenen Mitteln hergestellt, wobei das erste dieser beiden Mittel ein Gemisch aus alkoxyliertem Fettsäureester (a3) und farbgebender Verbindung(en), insbesondere Pigment(en) (a2) umfasst. So kann

das Gemisch aus alkoxyliertem Fettsäureester (a3) und Pigment(en) (a2) beispielsweise eine Vordispersion darstellen, die in Form eines Konzentrats zur Verfügung gestellt wird. Das zweite Mittel enthält mindestens ein aminofunkionalisiertes Silikonpolymer (a1) und kann beispielsweise eine wasserhaltige, kosmetische Trägerformulierung darsellen. Es ist ebenfalls erfindungsgemäß, wenn das erste Mittel, welches das Gemisch aus alkoxyliertem Fettsäureester (a3) und Pigment(en) (a2) umfasst, eine wasserhaltige kosmetische Trägerformulierung darstellt und das zweite Mittel, welches das aminofunkionalisiertes Silikonpolymer (a1) beinhaltet, in Form eines Konzentrats vorliegt. Zur Herstellung des anwendungsbereiten Mittes werden die beiden vorgenannten Mittel dann miteinander verschüttelt oder verrührt.

Weiterhin ist es erfindungsgemäß, dass anwendungsbereite Färbemittel durch vermischen von drei zuvor getrennt konfektionierten Mitteln herzustellen, wobei es sich bei dem ersten Mittel umd eine Mischung oder Vordisperion aus alkoxyliertem Fettsäureester (a3) und Pigment(en) (a2) handelt, das zweite Mischung ein aminofunkionalisiertes Silikonpolymer (a1) umfasst und beispielsweise in Form eines Konzentrats vorliegen kann, und das dritte Mittel eine wasserhaltige kosmetische Trägerformulierung darstellt, die bevorzugt mindestens ein alkoxliertes Alkanol (a4) enthält.

Ein weiterer Gegenstand der vorliegenden Anmeldung ist daher ein Verfahren zum Färben von keratinischem Material, insbesondere menschlichen Haaren, umfassend die folgenden Schritte:

(1) Bereitstellung eines Mittels (I), wobei das Mittel (I) enthält:

(a2) mindestens ein Pigment und
(a3) mindetens ein Anlagerungsprodukt von $C_1$-$C_6$-Alkylenoxid(en) an die Ester aus $C_{12}$-$C_{30}$-Fettsäuren und aromatischen $C_1$-$C_{12}$-Alkoholen,

(2) Bereitstellung eines Mittels (II), wobei das Mittel (II) enthält:
(a1) mindestens ein aminofunktionalisiertes Silikonpolymer, und
(3) Herstellung einer Anwendungsmischung durch Vermischen des Mittels (I) mit dem Mittel (II),
(4) Applizieren der in Schritt (3) hergestellten Anwendungsmischung auf dem keratinischen Material,
(5) Einwirken der in Schritt (4) applizierten Anwendungsmischung auf dem keratinischen Material und
(6) Ausspülen der Anwendungsmischung mit Wasser,

wobei die Inhaltsstoffe (a1), (a2) und (a3) bei der Beschreibung des ersten Erfindungsgegenstands bereits im Detail offenbart wurden.

Die optional zusätzlich anwendbaren alkoxylierten Alkanole (a4) können im Rahmen dieser Ausführungsform beispielsweise im Mittel (I) und/oder im Mittel (II) enthalten sein.

Abhängig vom gewählten pH-Wert hat das Aminosilikon (a3) in eingen Fällen eine verringerte Lagerstabilität in wässrigem Milieu gezeigt. In diesen Fällen kann es von Vorteil sein, auch das aminofunktionalisiertes Silikonpolymer (a3) in einem separaten Mittel zu konfektionieren und sowohl die Inhaltssoffe (a1) und (a2) als auch das Aminosilikon (a3) erst kurz vor der Anwendung mit einer Basis-Formulierung zu vermischen. In diesem Fall werden zur Herstellung des anwendungsbereiten Färbemittels mindestens drei verschiedene Mittel miteinander vermischt.

Besonders bevorzugt ist daher ein Verfahren zum Färben von keratinischem Material, insbesondere menschlichen Haaren, umfassend die folgenden Schritte:

(1) Bereitstellung eines Mittels (I), wobei das Mittel (I) enthält:

(a2) mindestens ein Pigment und
(a3) mindetens ein Anlagerungsprodukt von $C_1$-$C_6$-Alkylenoxid(en) an die Ester aus $C_{12}$-$C_{30}$-Fettsäuren und aromatischen $C_1$-$C_{12}$-Alkoholen,

(2) Bereitstellung eines Mittels (II), wobei das Mittel (II) enthält:
(a1) mindestens ein aminofunktionalisiertes Silikonpolymer, und
(3) Bereitstellung eines Mittels (III), wobei das Mittel (III) enhält:
(a4) mindestens ein Anlagerungsprodukt von $C_1$-$C_6$-Alkylenoxid(en) an aliphatische $C_1$-$C_{24}$-Alkanole,
(4) Herstellung einer Anwendungsmischung durch Vermischen des Mittels (I) mit dem Mittel (II) mit dem Mittel (III),
(5) Applizieren der in Schritt (4) hergestellten Anwendungsmischung auf dem keratinischen Material,
(6) Einwirken der in Schritt (5) applizierten Anwendungsmischung auf dem keratinischen Material und
(7) Ausspülen der Anwendungsmischung,

wobei die Inhaltsstoffe (a1), (a2), (a3) und (a4) bei der Beschreibung des ersten Erfindungsgegenstands bereits im Detail offenbart wurden.

Im Rahmen dieser Ausführungsform stellt das Mittel (I) bevorzugt eine Vordispersion der Pigmente (a2) in dem

oder den alkoxyliertem Fettsäureester (a3) dar, die beispielsweise in Form eines Konzentrats vorliegen kann. Das Mittel (II) ist bevorzugt ebenfalls ein Konzentrat, welches das oder die amino-funktionalisierten Silikonpolymere (a1) enthält.

Vor der Anwendung werden dann die beiden Mittel bzw. Konzentrate (I) und (II) mit der Trägerformulierung (III) vermischt. Die kosmetische Träger-Formulierung bzw. das Mittel (III), enthält im Rahmen dieser Ausführungsform die alkoxylierten Alkanole (a4).

Die Reihenfolge des Vermischens ist hierbei beliebig. So können zunächst die Mittel (I) und (II) minteinander vermischt werden, woraufhin diese Mischung dann mit dem Mittel (III) vermischt wird. Ebenso ist es denkbar, zunächst die Mittel (II) und (III) zu vermischen und diese Mischung dann mit dem Mittel (I) zu vermischen. Auch können alle drei Mittel (I), (II) und (III) zusammengegeben und denn erst durch Schütteln oder Rühren vermischt werden.

Die Trägerformulierung ist wasserhaltig und besitzt bevorzugt einen hohen Wassergehalt. Die optional einsatzbaren weiteren Inhaltsstoffe des ersten Erfindungsgegenstands können auch in dieser Trägerformulierung enthalten sein.

Betreffend die weiteren bevorzugten Ausführungsformen der erfindungsgemäßen Verfahren gilt *mutatis mutantis* das zum erfindungsgemäßen Mittel gesagte.

Beispiele

1. Formulierungen

Es wurden die folgenden Formulierungen hergestellt (alle Angaben, sofern nichts anderes angegeben ist, in Gew.-%), wobei Beispiele 1 und 2 nicht erfindungsgemäss sind:

| | | Bsp. 1 | Bsp. 2 | Bsp. 3 | Bsp. 4 |
|---|---|---|---|---|---|
| Creme Basis | Cetyl alcohol | 3,57 | 3,57 | 3,57 | 3,57 |
| | Stearyl alcohol | 1,98 | 1,98 | 1,98 | 1,98 |
| | Paraffinum Liquidum | 2,08 | 2,08 | 2,08 | 2,08 |
| | Ceteareth-30 | 1,19 | 1,19 | 1,19 | 1,19 |
| | Ceteareth-100 | 0,59 | 0,59 | 0,59 | 0,59 |
| | Glycerylstearat | 0,59 | 0,59 | 0,59 | 0,59 |
| | Water, dest. | 61,67 | 61,67 | 61,67 | 61,67 |
| | Kaliumdihydrogenphosphat | 0,35 | 0,35 | 0,35 | 0,35 |
| | Dinatriumhydrogenphosphat (Dihydrat) | 0,72 | 0,72 | 0,72 | 0,72 |
| | Phenoxyethanol | 0,89 | 0,89 | 0,89 | 0,89 |
| Predispersion | Unipure Red LC 3079 | 0,50 | --- | 0,50 | 0,38 |
| | PPG-3 Benzyl Ether Myristat Crodamol STS-LQ-(MH) | 4,95 | --- | 2,00 | 1,50 |
| Additive | Water, demineralized | 19,92 | 21,87 | 21,87 | 22,74 |
| | PPG-3 Benzyl Ether Myristat Crodamol STS-LQ-(MH) | --- | 3,00 | --- | --- |
| | Unipure Red LC 3079 | --- | 0,50 | --- | --- |
| | PPG-14 Butyl ether UCON FLUID AP | --- | --- | 1,00 | 1,00 |
| Aktivator | DOWSIL AP-8568 Amino Fluid, Aminosilikon | 1,00 | 1,00 | 1,00 | 0,75 |

Aus den angegebenen Bestandteilen wurde zunächst eine Cremebasis hergestellt. Zu dieser Cremebasis wurden dann die angegebenen Additive hinzugefügt.

Bei der Rezeptur des Beispiels 1 wurde als Additiv nur ein Anteil an Wasser zu der Cremebasis hinzugefügt.

Bei der Rezeptur des Beispiels 2 wurden als Additive Wasser, PPG-3 Benzyl Ether Myristat (a3) und das Pigment Unipure Red LC 3079 (a2) sukzessive in die Cremebasis eingearbeitet.

Bei den Rezepturen der Beispiele 3 und 4 wurden als Additive Wasser sowie PPG-14 Butyl ether (a4) zu der Cremebasis hinzugefügt.

Bei den Beispielrezepturen 1, 3 und 4 wurden dann jeweils das Pigment Unipure Red LC 3079 (a2) und PPG-3 Benzyl Ether Myristat (a3) separat vermischt, so dass dieses Gemisch in Form einer Predispersion vorlag. Diese

Predispersion wurde dann in die Cremebasis eingearbeitet.

Im letzten Schritt wurde bei allen vier Beispielrezepturen 1 bis 4 das Aminosilikon DOWSIL AP-8568 Amino Fluid (a1) hinzugefügt.

2. Anwendung

Haarsträhnen (Kerling Euronaturhaar weiß) wurden zunächst farbmetrisch mit einem Farbmessgerät der Firma Datacolor, Typ Spectraflash 450 vermessen.

Die Formulierungen der Beispiele 1 bis 4 wurden als Mittel zum Färben von Haaren wie folgt angewendet: Nach der Herstellung wurde das jeweilige Mittel auf Haarsträhnen (Kerling, Euronaturhaar weiß, Flottenverhältnis: 1 g Mittel (E1) pro g Haarsträhne) appliziert. Das Mittel wurde für drei Minuten einwirken gelassen. Im Anschluss daran wurden die Haarsträhne gründlich (1 Minute) mit Wasser ausgewaschen, getrocknet und dann erneut farbmetrisch mit einem Farbmessgerät der Firma Datacolor, Typ Spectraflash 450 vermessen.

Der für die Beurteilung der Farbintensität herangezogene ΔE-Wert ergibt sich aus den am jeweiligen Strähnenteil gemessenen L*a*b*-Farbmesswerten wie folgt:

$$\Delta E = [\ (L_i - L_0)^2 + (a_i - a_0)^2 + (b_i - b_0)]^{1/2}$$

$L_0$, $a_0$ und $b_0$ = Messwerte der Strähne vor der Färbung
$L_i$, $a_i$ und $b_i$ = Messwerte der Strähne nach der Färbung
Je größer dieser ΔE-Wert ist, desto höher (= besser) ist die Intensität der Färbung

Die gefärbten Haarsträhnen wurden im Hinblick auf ihren Griff von jeweils 10 geschulten Personen bewertet. Für das Haargefühl wurden Noten von 1 bis 6 verteilt (1: sehr schlechtes Haargefühl (rauh, spröde, stumpf), 6: sehr gutes Haargefühl (glatt, weich, gepflegt)).

Die gefärbten Haarsträhnen wurden auch im Hinblick auf ihre Reibechtheit bewertet. Für die Beurteiliung der Reibechtheit wurden Haarsträhnen einem standardisierten mechanischen Abrieb unterzogen und danach nochmals farbmetrisch vermessen. Der für die Beurteilung der Reibechtheit herangezogene ΔE-Wert ergibt sich aus den am jeweiligen Strähnenteil gemessenen L*a*b*-Farbmesswerten wie folgt:

$$\Delta E = [\ (L_i - L_0)^2 + (a_i - a_0)^2 + (b_i - b_0)]^{1/2}$$

$L_0$, $a_0$ und $b_0$ = Messwerte der Strähne nach der Färbung
$L_i$, $a_i$ und $b_i$ = Messwerte der Strähne nach der Färbung und nach mechanischer Beanspruchung Je größer dieser ΔE-Wert ist, desto höher ist die durch den Abrieb hervorgerufene Farbverschiebung, d.h. desto schlechter ist die Reibechtheit.

Nach dem Färben und Trocknen wurde jede Strähne 3 manuellen Haarwäschen unterzogen. Für jede Haarwäsche wurde die Strähne angefeuchtet, dann wurde ein handelsübliches Shampoo (Schwarzkopf, Schauma 7 Kräuter) 25 Sekunden lang in die Strähne einmassiert (0,25 g Shampoo pro Gramm Haar). Im Anschluss daran wurde die Strähne 30 Sekunden lang mit lauwarmem Leitungswasser ausgewaschen und getrocknet. Nach Abschluss der 3 Haarwäschen wurde jede Strähne erneut farbmetrisch vermessen.

Der für die Beurteilung der Waschechtheit herangezogene ΔE-Wert ergibt sich aus den gemessenen L*a*b*-Farbmesswerten wie folgt:

$$\Delta E = [\ (L_i - L_0)^2 + (a_i - a_0)^2 + (b_i - b_0)]^{1/2}$$

$L_0$, $a_0$ und $b_0$ = Messwerte der gefärbten Strähne nach dem Färben und vor dem Waschen
$L_i$, $a_i$ und $b_i$ = Messwerte der gefärbten Strähne nach 3 Haarwäschen
Je größer dieser ΔE-Wert ist, desto höher ist die durch die Wäsche hervorgerufene Farbverschiebung, d.h. desto schlechter ist die Waschechtheit.

Es wurden die folgendne Ergebnisse erhalten:

| | Bsp. 1 | Bsp. 2 | Bsp. 3 | Bsp. 4 |
|---|---|---|---|---|
| Farbintensität, ΔE-Wert (vor der Färbung - nach der Färbung) | 67 | 69 | 69 | 67 |

(fortgesetzt)

| | Bsp. 1 | Bsp. 2 | Bsp. 3 | Bsp. 4 |
|---|---|---|---|---|
| Waschechtheit, ΔE-Wert (vor der Wäsche - nach 3 Haarwäschen) | 14 | 19 | 14 | 11 |
| Reibechtheit, ΔE-Wert (vor dem Abrieb - nach dem Abrieb) | 11 | 18 | 8 | 7 |
| Haargefühl / Haargriff | 2 | 3 | 2,2 | 2,2 |

## Patentansprüche

**1.** Mittel zum Färben von keratinischem Material, insbesondere menschlichen Haaren, enthaltend

(a1) mindestens ein aminofunktionalisiertes Silikonpolymer, und
(a2) mindestens eine farbgebende Verbindung, und
(a3) mindestens ein Anlagerungsprodukt von $C_1$-$C_6$-Alkylenoxid(en) an die Ester aus $C_{12}$-$C_{30}$-Fettsäuren und aromatischen $C_1$-$C_{12}$-Alkoholen, und
(a4) mindestens ein Anlagerungsprodukt von $C_1$-$C_6$-Alkylenoxid(en) an aliphatische $C_1$-$C_{24}$-Alkanole der Formel (AA-I),

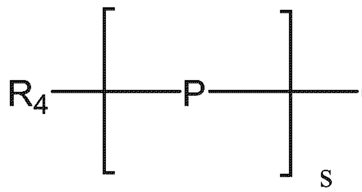

(AA-I)

worin

R4 für eine gesättigte oder ungesättigte $C_1$-$C_{12}$-Alkylgruppe steht, und
P für eine Struktureinheit $-O-CH_2-CH_2-$ , $-O-CH(CH_3)-CH_2-$ oder $-O-CH_2-CH(CH_3)-$ steht, und
s für eine ganze Zahl von 1 bis 60 steht.

**2.** Mittel nach Anspruch 1, **dadurch gekennzeichnet, dass** es mindestens ein aminofunktionalisiertes Silikonpolymer (a1) mit mindestens einer sekundären Aminogruppe enthält.

**3.** Mittel nach einem der Ansprüche 1 bis 2 **dadurch gekennzeichnet, dass** es mindestens ein aminofunktionalisiertes Silikonpolymer (a1) enthält, das mindestens eine Struktureinheit der Formel (Si-Amino) umfasst,

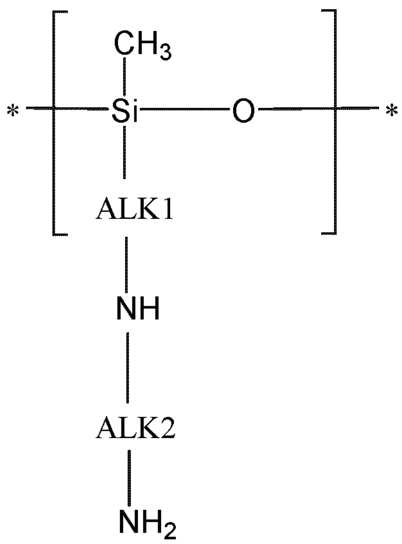

(Si-Amino)

wobei

ALK1 und ALK2 unabhängig voneinander für eine lineare oder verzweigte, zweiwertige $C_1$-$C_{20}$-Alkylengruppe stehen.

**4.** Mittel nach einem der Ansprüche 1 bis 3, **dadurch gekennzeichnet, dass** es mindestens ein aminofunktionalisiertes Silikonpolymer (a1) enthält, das Struktureinheiten der Formel (Si-I) und der Formel (Si-II) umfasst

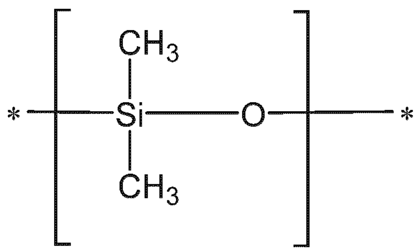
(Si-I)

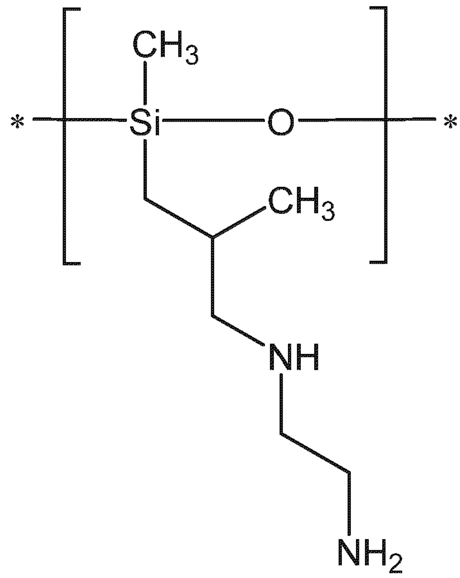
(Si-II).

**5.** Mittel nach einem der Ansprüche 1 bis 4, **dadurch gekennzeichnet, dass** es - bezogen auf das Gesamtgewicht des Mittels - ein oder mehrere aminofunktionalisierte Silikonpolymere (a1) in einer Gesamtmenge von 0,1 bis 8,0 Gew.-%, bevorzugt 0,2 bis 5,0 Gew.-%, weiter bevorzugt von 0,3 bis 3,0 Gew.-% und ganz besonders bevorzugt von 0,4 bis 2,5 Gew.-% enthält.

**6.** Mittel nach einem der Ansprüche 1 bis 5, **dadurch gekennzeichnet, dass** es mindestens eine farbgebende Verbindung (a2) aus der Gruppe der Pigmente, der direktziehenden Farbstoffe, der photochromen Farbstoffe und der thermochromen Farbstoffe enthält.

**7.** Mittel nach einem der Ansprüche 1 bis 6, **dadurch gekennzeichnet, dass** es mindestens eine farbgebende Verbindung (a2) aus der Gruppe der anorganischen Pigmente enthält, die bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

**8.** Mittel nach einem der Ansprüche 1 bis 7, **dadurch gekennzeichnet, dass** es mindestens eine farbgebende Verbindung (a2) aus der Gruppe der organischen Pigmente enthält, die bevorzugt ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

**9.** Mittel nach einem der Ansprüche 1 bis 8, **dadurch gekennzeichnet, dass** es - bezogen auf das Gesamtgewicht des Mittels (a) - ein odere mehrere Pigmente (a2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthält.

**10.** Mittel nach einem der Ansprüche 1 bis 9, **dadurch gekennzeichnet, dass** es

(a3) mindestens ein Anlagerungsprodukt von $C_1$-$C_6$-Alkylenoxid(en) an einen Ester enthält, der durch Veresterung von einer $C_{12}$-$C_{24}$-Fettsäure mit einem aromatischen $C_1$-$C_{12}$-Alkohol aus der Gruppe aus Benzylalkohol, Phenol, 2-Phenylethylalkohol und 2-Phenoxyethanol, ganz besonders bevorzugt Benzylalkohol, erhalten wird.

**11.** Mittel nach einem der Ansprüche 1 bis 10, **dadurch gekennzeichnet, dass** es
(a3) mindestens einen alkoxylierten Fettsäureester der allgemeinen Formel (AFE-I) enthält

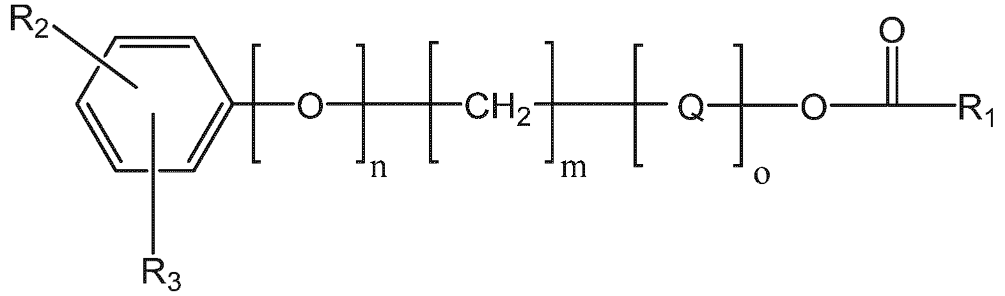

(AFE-I)

wobei

R1 für eine gesättigte oder ungesättige $C_{11}$-$C_{29}$-Alkylgruppe steht
R2, R3 unabhängig voneinander für ein Wasserstoffatom, eine $C_1$-$C_6$-Alkylgruppe, eine Hydroxygruppe oder eine $C_1$-$C_6$-Alkoxygruppe stehen,
n für die Zahl 0 oder 1 steht,
m für eine ganze Zahl von 0 bis 6 steht,
o für eine ganze Zahl von 1 bis 60 steht, und
Q für eine Struktureinheit $-O-CH_2-CH_2-$ , $-O-CH(CH_3)-CH_2-$ oder $-O-CH_2-CH(CH_3)-$steht,

wobei die Maßgabe besteht, dass, wenn m gleich 0 ist, auch n gleich 0 ist.

**12.** Mittel nach Anspruch 11, **dadurch gekennzeichnet, dass** es
(a3) mindestens einen alkoxylierten Fettsäureester der allgemeinen Formel (AFE-I) enthält wobei

R1 für eine gesättigte oder ungesättige $C_{11}$-$C_{29}$-Alkylgruppe steht
R2, R3 beide für ein Wasserstoffatom stehen,
n für die Zahl 0 steht,
m für die Zahl 1 steht,
o für eine ganze Zahl von 1 bis 30, bevorzugt von 1 bis 20, weiter bevorzugt von 1 bis 10 und ganz besonders bevorzugt von 1 bis 5 steht, und
Q für eine Struktureinheit $-O-CH(CH_3)-CH_2-$ oder $-O-CH_2-CH(CH_3)-$ steht.

**13.** Mittel nach einem der Ansprüche 1 bis 12, **dadurch gekennzeichnet, dass** es - bezogen auf das Gesamtgewicht des Mittels - ein oder mehrere alkoxylierte Fettsäureester (a3) in einer Gesamtmenge von 0,1 bis 20,0 Gew.-%, bevorzugt 0,5 bis 15,0 Gew.-%, weiter bevorzugt von 1,0 bis 10,0 Gew.-%, noch weiter bevorzugt von 1,0 bis 6,0 Gew.-% und ganz besonders bevorzugt von 1,0 bis 1,8 Gew.-% enthält.

**14.** Mittel nach einem der Ansprüche 1 bis 13, dadurch gekennzeichet, dass es
(a4) mindestens ein Anlagerungsprodukt von $C_1$-$C_6$-Alkylenoxid(en) an aliphatische $C_1$-$C_{24}$-Alkanole der Formel (AA-I) enthält,

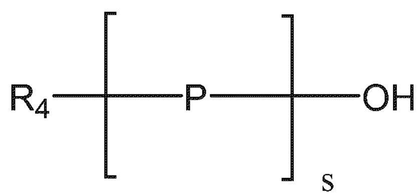

(AA-I)

worin

R4 für eine gesättigte oder ungesättigte $C_1$-$C_6$-Alkylgruppe steht, und
P für eine Struktureinheit -O-$CH_2$-$CH_2$- , -O-CH($CH_3$)-$CH_2$- oder -O-$CH_2$-CH($CH_3$)- steht, und
s für eine ganze Zahl von 1 bis 60, bevorzugt für eine ganze Zahl von 1 bis 40, weiter bevorzugt für eine ganze Zahl von 10 bis 30 und ganz besonders bevorzugt für eine ganze Zahl von 10 bis 20, steht.

**15.** Mittel nach einem der Ansprüche 1 bis 14, **dadurch gekennzeichnet, dass** es - bezogen auf das Gesamtgewicht des Mittels - ein oder mehrere Anlagerungsprodukte von $C_1$-$C_6$-Alkylenoxid(en) an aliphatische $C_1$-$C_{24}$-Alkanole (a4) in einer Gesamtmenge von 0,1 bis 20,0 Gew.-%, bevorzugt 0,2 bis 15,0 Gew.-%, weiter bevorzugt von 0,3 bis 10,0 Gew.-%, noch weiter bevorzugt von 0,4 bis 5,0 Gew.-%, noch weite bevorzugt von 0,5 bis 3,0 Gew.-% und ganz besonders bevorzugt von 0,5 bis 1,5 Gew.-% enthält.

**16.** Mittel nach einem der Ansprüche 1 bis 15, **dadurch gekennzeichnet, dass** es Wasser enthält und einen pH-Wert von 3,8 bis 11,5 bevorzugt von 4,0 bis 10,0, weiter bevorzugt von 5,0 bis 9,0 und ganz besonders bevorzugt von 6,0 bis 8,0 besitzt.

**17.** Verfahren zum Färben von keratinischem Material, insbesondere menschlichen Haaren, umfassend die folgenden Schritte:

(1) Anwendung eines Färbemittels nach einem der Ansprüche 1 bis 16 auf dem keratinischem Material,
(2) Einwirken des Färbemittels auf dem keratinischen Material und
(3) Ausspülen des Färbemittels.

## Claims

**1.** An agent for dyeing keratinous material, in particular human hair, containing

(a1) at least one amino-functionalized silicone polymer, and
(a2) at least one coloring compound, and
(a3) at least one addition product of $C_1$-$C_6$ alkylene oxide(s) to the esters of $C_{12}$-$C_{30}$ fatty acids and aromatic $C_1$-$C_{12}$ alcohols, and
(a4) at least one addition product of $C_1$-$C_6$ alkylene oxide(s) to aliphatic $C_1$-$C_{24}$ alkanols of formula (AA-I),

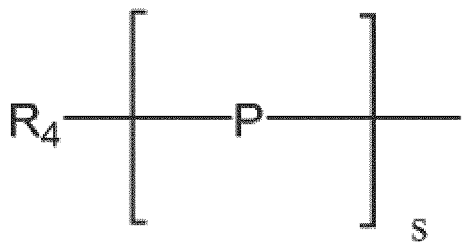

(AA-I)

where

R4 represents a saturated or unsaturated $C_1$-$C_{12}$ alkyl group, and
P represents a structural unit -Q-$CH_2$-$CH_2$-, -O-CH($CH_3$)-$CH_2$- or -O-$CH_2$-CH($CH_3$)-, and
s represents an integer from 1 to 60.

**2.** The agent according to claim 1, **characterized in that** it contains at least one amino-functionalized silicone polymer (a1) having at least one secondary amino group.

**3.** The agent according to one of claims 1 to 2, **characterized in that** it contains at least one amino-functionalized silicone polymer (a1) which comprises at least one structural unit of the formula (Si-amino),

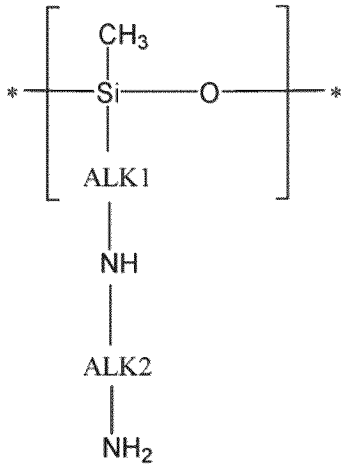

(Si-amino)

where

ALK1 and ALK2 represent, independently of one another, a linear or branched, divalent $C_1$-$C_{20}$ alkylene group.

4. The agent according to one of claims 1 to 3, **characterized in that** it contains at least one amino-functionalized silicone polymer (a1) which comprises structural units of formula (Si-I) and formula (Si-II)

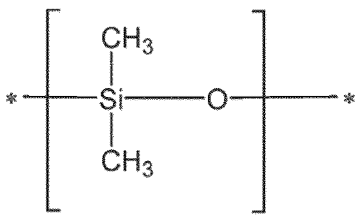

(Si-I)

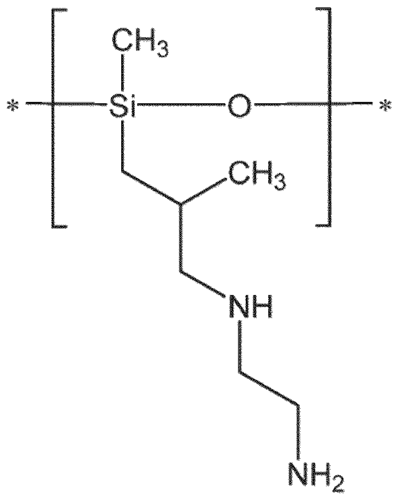

(Si-II).

5. The agent according to one of claims 1 to 4, **characterized in that** it contains, based on the total weight of the agent, one or more amino-functionalized silicone polymers (a1) in a total amount from 0.1 to 8.0 wt.%, preferably from 0.2 to 5.0 wt.%, more preferably from 0.3 to 3.0 wt.%, and very particularly preferably from 0.4 to 2.5 wt.%.

6. The agent according to one of claims 1 to 5, **characterized in that** it contains at least one coloring compound (a2) from the group of pigments, direct dyes, photochromic dyes and thermochromic dyes.

7. The agent according to one of claims 1 to 6, **characterized in that** it contains at least one coloring compound (a2) from the group of inorganic pigments, preferably selected from the group of colored metal oxides, metal hydroxides, metal oxide hydrates, silicates, metal sulfides, complex metal cyanides, metal sulphates, bronze pigments and/or from mica-based colored pigments which are coated with at least one metal oxide and/or a metal oxychloride.

8. The agent according to one of claims 1 to 7, **characterized in that** it contains at least one coloring compound (a2) from the group of organic pigments, preferably selected from the group of carmine, quinacridone, phthalocyanine, sorghum, blue pigments with the Color Index numbers CI 42090, CI 69800, CI 69825, CI 73000, CI 74100 or CI 74160, yellow pigments with the Color Index numbers CI 11680, CI 11710, CI 15985, CI 19140, CI 20040, CI 21100, CI 21108, CI 47000 or CI 47005, green pigments with the Color Index numbers CI 61565, CI 61570 or CI 74260, orange pigments with the Color Index numbers CI 11725, CI 15510, CI 45370 or CI 71105, red pigments with the Color Index

numbers CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 and/or CI 75470.

**9.** The agent according to one of claims 1 to 8, **characterized in that** it contains, based on the total weight of the agent (a), one or more pigments (a2) in a total amount from 0.01 to 10.0 wt.%, preferably from 0.1 to 5.0 wt.%, more preferably from 0.2 to 2.5 wt.%, and very particularly preferably from 0.25 to 1.5 wt.%.

**10.** The agent according to one of claims 1 to 9, **characterized in that** it contains
(a3) at least one addition product of $C_1$-$C_6$ alkylene oxide(s) to an ester obtained by esterification of a $C_{12}$-$C_{24}$ fatty acid with an aromatic $C_1$-$C_{12}$ alcohol from the group of benzyl alcohol, phenol, 2-phenylethyl alcohol and 2-phenoxyethanol, very particularly preferably benzyl alcohol.

**11.** The agent according to one of claims 1 to 10, **characterized in that** it contains
(a3) at least one alkoxylated fatty acid ester of general formula (AFE-I)

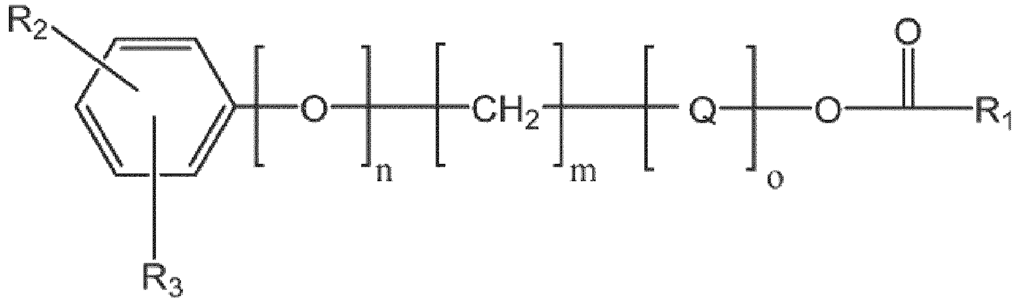

(AFE-I)

where

R1 represents a saturated or unsaturated $C_{11}$-$C_{29}$ alkyl group
R2, R3 represent, independently of one another, a hydrogen atom, a $C_1$-$C_6$ alkyl group, a hydroxy group, or a $C_1$-$C_6$ alkoxy group,
n represents the number 0 or 1,
m represents an integer from 0 to 6,
o represents an integer from 1 to 60, and
Q represents a structural unit $-Q-CH_2-CH_2-$, $-O-CH(CH_3)-CH_2-$ or $-O-CH_2-CH(CH3)-$,
with the proviso that if m is equal to 0, n is also equal to 0.

**12.** The agent according to claim 11, **characterized in that** it contains

(a3) at least one alkoxylated fatty acid ester of general formula (AFE-I), where
R1 represents a saturated or unsaturated $C_{11}$-$C_{29}$ alkyl group,
R2, R3 both represent a hydrogen atom,
n represents the number 0,
m represents the number 1,
o represents an integer from 1 to 30, preferably from 1 to 20, more preferably from 1 to 10, and very particularly preferably from 1 to 5, and
Q represents a structural unit $-Q-CH(CH_3)-CH_2-$ or $-O-CH_2-CH(CH_3)-$.

**13.** The agent according to one of claims 1 to 12, **characterized in that** it contains, based on the total weight of the agent, one or more alkoxylated fatty acid esters (a3) in a total amount from 0.1 to 20.0 wt.%, preferably from 0.5 to 15.0 wt.%, more preferably from 1.0 to 10.0 wt.%, even more preferably from 1.0 to 6.0 wt.%, and very particularly preferably from 1.0 to 1.8 wt.%.

**14.** The agent according to one of claims 1 to 13, **characterized in that** it contains
(a4) at least one addition product of $C_1$-$C_6$ alkylene oxide(s) to aliphatic $C_1$-$C_{24}$ alkanols of formula (AA-I),

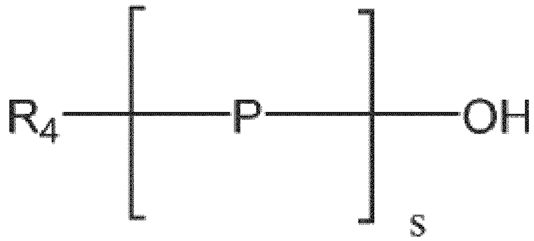

(AA-I)

where

R4 represents a saturated or unsaturated $C_1$-$C_6$ alkyl group, and
P represents a structural unit -Q-$CH_2$-$CH_2$-, -O-CH($CH_3$)-$CH_2$- or -O-$CH_2$-CH($CH_3$)-,
and
s represents an integer from 1 to 60, preferably an integer from 1 to 40, more preferably an integer from 10 to 30, and very particularly preferably an integer from 10 to 20.

**15.** The agent according to one of claims 1 to 14, **characterized in that** it contains, based on the total weight of the agent, one or more addition products of $C_1$-$C_6$ alkylene oxide(s) to aliphatic $C_1$-$C_{24}$ alkanols (a4) in a total amount from 0.1 to 20.0 wt.%, preferably from 0.2 to 15.0 wt.%, more preferably from 0.3 to 10.0 wt.%, even more preferably from 0.4 to 5.0 wt.%, even more preferably from 0.5 to 3.0 wt. %, and very particularly preferably from 0.5 to 1.5 wt.%.

**16.** The agent according to one of claims 1 to 15, **characterized in that** it contains water and has a pH from 3.8 to 11.5, preferably from 4.0 to 10.0, more preferably from 5.0 to 9.0, and very particularly preferably from 6.0 to 8.0.

**17.** A method for dyeing keratinous material, in particular human hair, comprising the following steps:

(1) applying a coloring agent according to one of claims 1 to 16 to the keratinous material,
(2) letting the coloring agent act on the keratinous material, and
(3) rinsing out the coloring agent.

**Revendications**

**1.** Agent permettant la coloration d'une matière kératinique, en particulier de cheveux humains, contenant

(a1) au moins un polymère de silicone aminofonctionnalisé, et
(a2) au moins un composé colorant, et
(a3) au moins un produit d'addition d'oxyde(s) d'alkylène en $C_1$-$C_6$ aux esters d'acides gras en $C_{12}$-$C_{30}$ et d'alcools aromatiques en $C_1$-$C_{12}$, et
(a4) au moins un produit d'addition d'oxyde(s) d'alkylène en $C_1$-$C_6$ à des alcanols aliphatiques en $C_1$-$C_{24}$ de formule (AA-I),

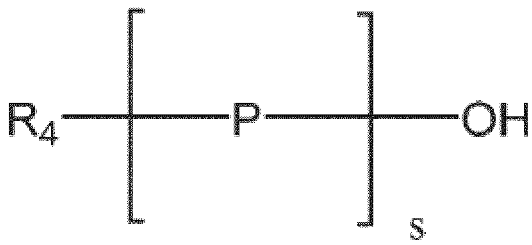

(AA-I)

où

R4 représente un groupe alkyle en $C_1$-$C_{12}$ saturé ou insaturé, et
P représente un motif structural -O-$CH_2$-$CH_2$-, -O-CH($CH_3$)-$CH_2$- ou -O-$CH_2$-CH($CH_3$)-,

et
s représente un nombre entier allant de 1 à 60.

2. Agent selon la revendication 1, **caractérisé en ce qu'**il contient au moins un polymère de silicone aminofonctionnalisé (a1) comportant au moins un groupe amine secondaire.

3. Agent selon l'une des revendications 1 à 2, **caractérisé en ce qu'**il contient au moins un polymère de silicone aminofonctionnalisé (a1) qui comprend au moins un motif structural de formule (Si-Amino),

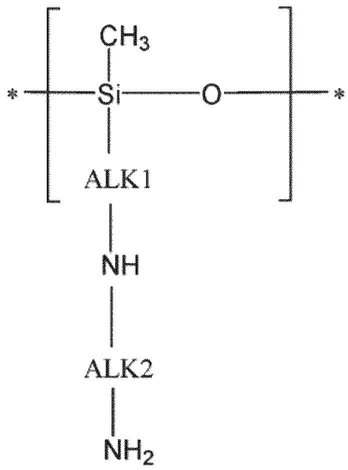

(Si-Amino)

où
ALK1 et ALK2 représentent, indépendamment l'un de l'autre, un groupe alkylène en $C_1$-$C_{20}$ bivalent, linéaire ou ramifié.

4. Agent selon l'une des revendications 1 à 3, **caractérisé en ce qu'**il contient au moins un polymère de silicone aminofonctionnalisé (a1) qui comprend des motifs structuraux de formule (Si-I) et de formule (Si-II)

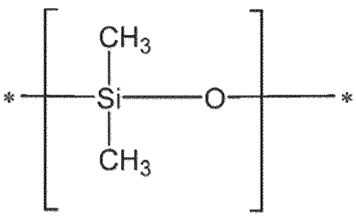

(Si-I)

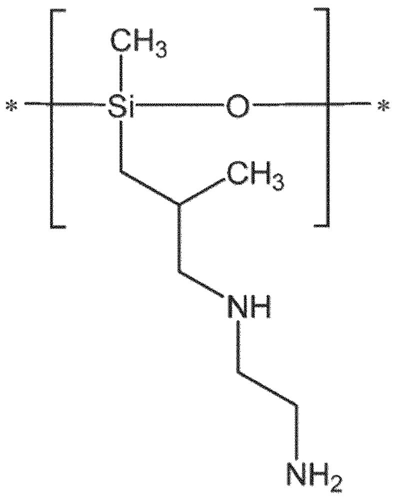

(Si-II).

5. Agent selon l'une des revendications 1 à 4, **caractérisé en ce qu'**il contient, par rapport au poids total de l'agent, un ou plusieurs polymères de silicone aminofonctionnalisés (a1) en une quantité totale allant de 0,1 à 8,0 % en poids, de préférence de 0,2 à 5,0 % en poids, plus préférablement de 0,3 à 3,0 % en poids et de manière tout particulièrement préférée de 0,4 à 2,5 % en poids.

6. Agent selon l'une des revendications 1 à 5, **caractérisé en ce qu'**il contient au moins un composé colorant (a2) du groupe constitué de pigments, colorants directs, colorants photochromiques et colorants thermochromiques.

7. Agent selon l'une des revendications 1 à 6, **caractérisé en ce qu'**il contient au moins un composé colorant (a2) du groupe des pigments inorganiques qui est de préférence choisi dans le groupe constitué d'oxydes métalliques colorés, hydroxydes métalliques colorés, oxydes métalliques hydratés colorés, silicates colorés, sulfures métalliques colorés, cyanures métalliques complexes colorés, sulfates métalliques colorés, pigments de bronze colorés et/ou de pigments colorés à base de mica recouverts d'au moins un oxyde métallique et/ou oxychlorure métallique.

8. Agent selon l'une des revendications 1 à 7, **caractérisé en ce qu'**il contient au moins un composé colorant (a2) du groupe des pigments organiques qui est de préférence choisi dans le groupe constitué de carmin, quinacridone, phtalocyanine, sorgho, pigments bleus comportant les numéros d'indice de couleur CI 42090, CI 69800, CI 69825, CI 73000, CI 74100, CI 74160, pigments jaunes comportant les numéros d'indice de couleur CI 11680, CI 11710, CI 15985, CI 19140, CI 20040, CI 21100, CI 21108, CI 47000, CI 47005, pigments verts comportant les numéros d'indice de couleur CI 61565, CI 61570, CI 74260, pigments orange comportant les numéros d'indice de couleur CI 11725, CI 15510, CI 45370, CI 71105, pigments rouges comportant les numéros d'indice de couleur CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 et/ou CI 75470.

9. Agent selon l'une des revendications 1 à 8, **caractérisé en ce qu'**il contient, par rapport au poids total de l'agent (a), un ou plusieurs pigments (a2) en une quantité totale allant de 0,01 à 10,0 % en poids, de préférence de 0,1 à 5,0 % en poids, plus préférablement de 0,2 à 2,5 % en poids et de manière tout particulièrement préférée de 0,25 à 1,5 % en poids.

10. Agent selon l'une des revendications 1 à 9, **caractérisé en ce qu'**il
(a3) contient au moins un produit d'addition d'oxyde(s) d'alkylène en $C_1$-$C_6$ à un ester obtenu par estérification d'un acide gras en $C_{12}$-$C_{24}$ avec un alcool aromatique en $C_1$-$C_{12}$ du groupe constitué d'alcool benzylique, phénol, alcool 2-phényléthylique et 2-phénoxyéthanol, de manière tout particulièrement préférée d'alcool benzylique.

11. Agent selon l'une des revendications 1 à 10, **caractérisé en ce qu'**il
(a3) contient au moins un ester d'acide gras alcooxylé de formule générale (AFE-I)

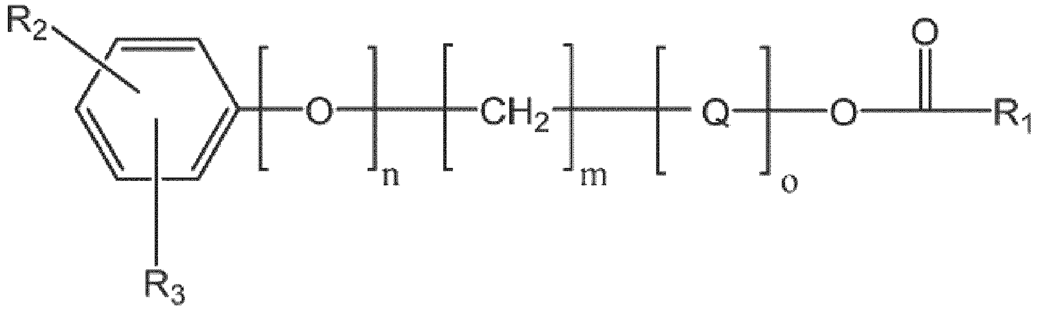

(AFE-I)

où

R1 représente un groupe alkyle en $C_{11}$-$C_{29}$ saturé ou insaturé,
R2, R3 représentent, indépendamment l'un de l'autre, un atome d'hydrogène, un groupe alkyle en $C_1$-$C_6$, un groupe hydroxy ou un groupe alcoxy en $C_1$-$C_6$,
n représente le nombre 0 ou 1,
m représente un nombre entier allant de 0 à 6,
o représente un nombre entier allant de 1 à 60, et
Q représente un motif structural $-O-CH_2-CH_2-$, $-O-CH(CH_3)-CH_2-$ ou $-O-CH_2-CH(CH_3)-$,
dans lequel, à condition que, si m est égal à 0, n soit également égal à 0.

12. Agent selon la revendication 11, **caractérisé en ce qu'**il

(a3) contient au moins un ester d'acide gras alcooxylé de formule générale (AFE-I), où
R1 représente un groupe alkyle en $C_{11}$-$C_{29}$ saturé ou insaturé
R2, R3 représentent tous deux un atome d'hydrogène,
n représente le nombre 0,

m représente le nombre 1,
o représente un nombre entier allant de 1 à 30, de préférence de 1 à 20, plus préférablement de 1 à 10 et de manière tout particulièrement préférée de 1 à 5, et
Q représente un motif structural $-O-CH(CH_3)-CH_2-$ ou $-O-CH_2-CH(CH_3)-$.

**13.** Agent selon l'une des revendications 1 à 12, **caractérisé en ce qu'**il contient, par rapport au poids total de l'agent, un ou plusieurs esters d'acides gras alcooxylés (a3) en une quantité totale allant de 0,1 à 20,0 % en poids, de préférence de 0,5 à 15,0 % en poids, plus préférablement de 1,0 à 10,0 % en poids, encore plus préférablement de 1,0 à 6,0 % en poids et de manière tout particulièrement préférée de 1,0 à 1,8 % en poids.

**14.** Agent selon l'une des revendications 1 à 13, **caractérisé en ce qu'**il
(a4) contient au moins un produit d'addition d'oxyde(s) d'alkylène en $C_1$-$C_6$ à des alcanols aliphatiques en $C_1$-$C_{24}$ de formule (AA-I),

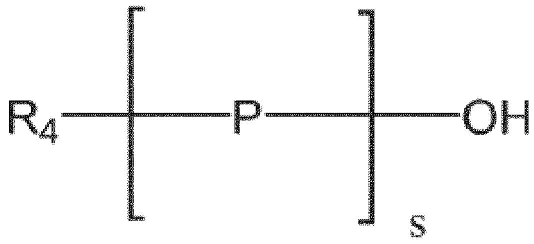

(AA-I)

où

R4 représente un groupe alkyle en $C_1$-$C_6$ saturé ou insaturé, et
P représente un motif structural $-O-CH_2-CH_2-$, $-O-CH(CH_3)-CH_2-$ ou $-O-CH_2-CH(CH_3)-$,
et
s représente un nombre entier allant de 1 à 60, de préférence un nombre entier allant de 1 à 40, plus préférablement un nombre entier allant de 10 à 30 et de manière tout particulièrement préférée un nombre entier allant de 10 à 20.

**15.** Agent selon l'une des revendications 1 à 14, **caractérisé en ce qu'**il contient, par rapport au poids total de l'agent, un ou plusieurs produits d'addition d'oxyde(s) d'alkylène en $C_1$-$C_6$ à des alcanols aliphatiques en $C_1$-$C_{24}$ (a4) en une quantité totale allant de 0,1 à 20,0 % en poids, de préférence de 0,2 à 15,0 % en poids, plus préférablement de 0,3 à 10,0 % en poids, encore plus préférablement de 0,4 à 5,0 % en poids, encore plus préférablement de 0,5 à 3,0 % en poids et de manière tout particulièrement préférée de 0,5 à 1,5 % en poids.

**16.** Agent selon l'une des revendications 1 à 15, **caractérisé en ce qu'**il contient de l'eau et possède un pH allant de 3,8 à 11,5, de préférence de 4,0 à 10,0, plus préférablement de 5,0 à 9,0 et de manière tout particulièrement préférée de 6,0 à 8,0.

**17.** Procédé permettant la coloration d'une matière kératinique, en particulier de cheveux humains, comprenant les étapes suivantes :

(1) application d'un agent de coloration selon l'une des revendications 1 à 16 sur la matière kératinique,
(2) attente de l'action de l'agent de coloration sur la matière kératinique et
(3) rinçage de l'agent de coloration.